(19) Europäisches Patentamt
European Patent Office
Office européen des brevets

(11) EP 4 722 215 A1

# (12) EUROPEAN PATENT APPLICATION
published in accordance with Art. 153(4) EPC

(43) Date of publication:
**08.04.2026 Bulletin 2026/15**

(21) Application number: **24810482.0**

(22) Date of filing: **24.05.2024**

(51) International Patent Classification (IPC):
***C07D 471/10*** (2006.01) ***A61K 31/497*** (2006.01)
***A61P 13/08*** (2006.01) ***A61P 35/00*** (2006.01)

(52) Cooperative Patent Classification (CPC):
**A61K 31/497; A61P 13/08; A61P 35/00; C07D 471/10**

(86) International application number:
**PCT/CN2024/095156**

(87) International publication number:
**WO 2024/240246 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.05.2023 CN 202310592195**

(71) Applicants:
- **Jiangsu Hengrui Pharmaceuticals Co., Ltd.**
**Lianyungang, Jiangsu 222047 (CN)**
- **Shanghai Hengrui Pharmaceutical Co., Ltd.**
**Shanghai 200245 (CN)**

(72) Inventors:
- **ZHOU, Xianqiang**
**Lianyungang, Jiangsu 222047 (CN)**
- **SHANG, Tingting**
**Lianyungang, Jiangsu 222047 (CN)**
- **WANG, Lin**
**Shanghai 200245 (CN)**
- **DU, Zhenxing**
**Lianyungang, Jiangsu 222047 (CN)**
- **YANG, Junran**
**Lianyungang, Jiangsu 222047 (CN)**

(74) Representative: **Dragotti & Associati S.P.A.**
**Via Nino Bixio, 7**
**20129 Milano (IT)**

## (54) AMORPHOUS SUBSTANCE AND CRYSTAL OF DIOXOPIPERIDINE COMPOUND OR PHARMACEUTICALLY ACCEPTABLE SALT THEREOF, PREPARATION METHOD, AND USE

(57) The present disclosure relates to an amorphous substance and a crystal of a dioxopiperidine compound or a pharmaceutically acceptable salt thereof, a preparation method, and a use. Specifically, the present disclosure relates to an amorphous substance and a crystal of a compound shown in formula I or a pharmaceutically acceptable salt thereof, which have good physical and chemical properties.

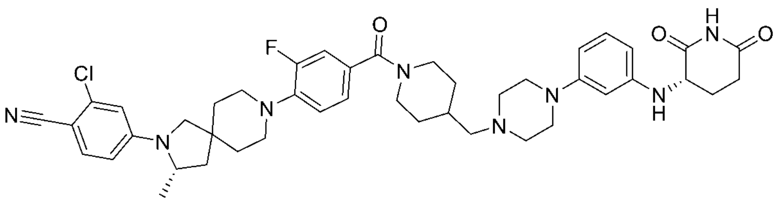

I

EP 4 722 215 A1

## Description

The present application claims priority to Chinese Patent Application No. 2023105921952 filed on May 24, 2023, which is incorporated herein by reference in its entirety.

## TECHNICAL FIELD

The present disclosure belongs to the field of pharmaceuticals, and relates to an amorphous form and a crystal of a dioxopiperidine compound or a pharmaceutically acceptable salt thereof, a preparation method therefor, and use thereof.

## BACKGROUND

PROTACs (PROteolysis TArgeting Chimeras) are hybrid bifunctional small-molecule compounds. Their structures contain two different ligands: a ubiquitin ligase E3 ligand and a ligand that binds to a target protein, which are linked by a linker unit. PROTACs brings the target protein and the intracellular ubiquitin ligase E3 into proximity to form a target protein-PROTAC-E3 ternary complex. Subsequently, the E3 ubiquitin ligase labels the target protein with a ubiquitinated protein tag and then initiates the powerful ubiquitination-proteasome system in the cell to specifically degrade the target protein, thereby playing a role in inhibiting the corresponding protein signaling pathway *(*Cell Biochem Funct., 2019, 37, 21-30). PROTACs have unique advantages over traditional small-molecule inhibitors: 1) PROTACs do not require long and high-strength binding to the target protein, and their degradation of the target protein is similar to catalysis: they can bind and degrade the target protein cyclically, so that the systemic drug exposure and the occurrence of toxic and side effects are reduced; 2) after being degraded, the target protein needs to be re-synthesized to recover its function; therefore, degrading the target protein exhibits a more efficient and lasting anti-tumor effect than inhibiting its activity and will not lead to drug resistance caused by mutation of the target protein; 3) PROTACs also have therapeutic potentials for the targets which are now considered undruggable, such as transcription factors, scaffold proteins, and regulatory proteins.

The discovery of CRBN-type E3 ligase ligands is related to the study of the mechanism of action of thalidomide. In 2010, scientists discovered cereblon, a binding protein of thalidomide, while studying the toxicity of thalidomide (Science 2010, 327, 1345). Cereblon is part of the E3 ubiquitin ligase protein complex. As a substrate receptor, it selectively acts on ubiquitinated proteins. This study indicated that the in vivo binding of thalidomide to cereblon might be the cause of thalidomide's teratogenicity. Subsequent studies found that this compound and related structures could be used as anti-inflammatory agents, anti-angiogenesis agents, and anti-cancer agents. Lenalidomide and pomalidomide obtained by further modifying the thalidomide structure are much safer, and their teratogenic effects are significantly smaller. Lenalidomide was approved for sale by the FDA in 2006. In 2014, two groundbreaking papers published in Science pointed out that lenalidomide acts by degrading two special B-cell transcription factors, Ikaros family zinc finger structure proteins 1 and 3 (IKZF1 and IKZF3), which further reveals that the thalidomide structure may bind to the E3 ubiquitin ligase protein complex of cereblon, thereby playing a role in degrading the target protein (Science, 2014, 343, 301; Science, 2014, 343, 305).

The androgen receptor (AR) is a ligand-dependent transcriptional regulatory protein that belongs to the nuclear receptor superfamily, mainly found in the nucleus. AR molecules that are not bound by the ligand bind to the heat shock protein (HSP); however, upon binding to the ligand, the AR undergoes a conformational change and dissociates from the HSP, and its affinity for DNA increases (activation of the AR). Activated AR molecules bind in dimer form to a specific DNA sequence in the nucleus-the androgen response element (ARE)-and interact with other transcription factors, thereby regulating the expression of relevant genes and producing biological effects. Studies have shown that abnormalities in the AR signaling pathway are closely related to the development and progression of diseases such as prostate cancer, benign prostatic hyperplasia, Kennedy's disease, male infertility, androgen insensitivity syndrome, and male breast cancer. Prostate cancer is one of the most common malignancies. According to statistics, in 2018, there were nearly 1.3 million new cases and 359 thousand deaths worldwide, accounting for 13.5% of the incidence rate of malignancies in men, ranking second, and 6.7% of the mortality rate of malignancies in men, ranking fifth. A number of AR antagonists have been approved for sale and successfully used in the treatment of castration-resistant prostate cancer, and they have become a major treatment for prostate cancer. However, most patients develop resistance after 0.5-2 years of treatment, which leads to disease progression. In some patients with resistance, cancer cell growth still depends on the AR signaling pathway.

PCT/CN2022/134333 provides a compound for regulating the ubiquitination and degradation of the androgen receptor (AR) protein, which is chemically named 2-chloro-4-((S)-8-(4-(4-((4-(3-(((S)-2,6-dioxopiperidin-3-yl)amino) phenyl)piperazin-1-yl)methyl)piperidine-1-carbonyl)-2-fluorophenyl)-3-methyl-2,8-diazaspiro[4.5]decan-2-yl)benzonitrile, having the structure represented by formula I. This application is incorporated herein by reference in its entirety.

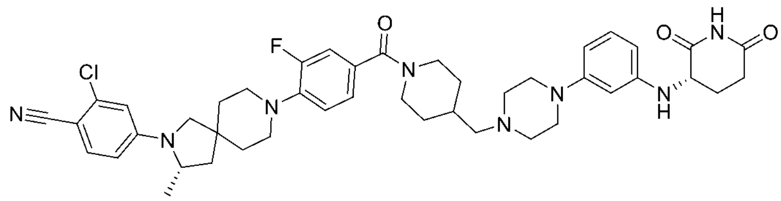

I

## SUMMARY

In a first aspect, the present disclosure provides an amorphous form of the compound represented by formula I.

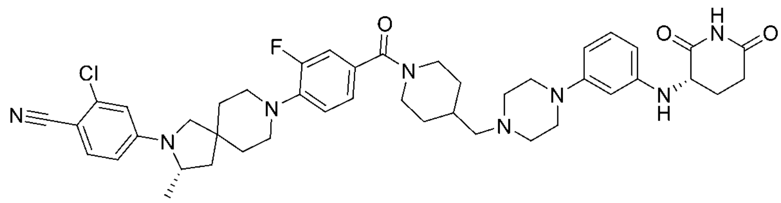

I

In some embodiments, an X-ray powder diffraction pattern of the amorphous form of the compound represented by formula I has no sharp peaks and preferably has non-sharp peaks; more preferably, the X-ray powder diffraction pattern of the amorphous form is shown in FIG. 1.

In some embodiments, a DSC thermogram of the amorphous form of the compound represented by formula I shows an endothermic peak at 122.02±3 °C.

In some embodiments, a thermogravimetric analysis (TGA) profile of the amorphous form of the compound represented by formula I shows a weight loss of about 5.06% from 33 °C to 170 °C.

The present disclosure further provides a preparation method for the amorphous form of the compound represented by formula I, comprising: mixing the compound represented by formula I with a solvent A, and crystallizing to give the amorphous form. In some embodiments, the solvent A is selected from the group consisting of one or more of water, methanol, ethanol, isopropanol, n-propanol, isopentanol, isopropyl acetate, methyl tert-butyl ether, n-heptane, cyclohexane, and toluene.

The present disclosure further provides a preparation method for the amorphous form of the compound represented by formula I, comprising: mixing the compound represented by formula I with a solvent B, adding an anti-solvent, and crystallizing to give the amorphous form. In some embodiments, the solvent B is selected from the group consisting of one or more of dimethyl sulfoxide, acetone, acetonitrile, tetrahydrofuran, and dichloromethane; the anti-solvent is selected from the group consisting of one or more of water, ethanol, n-heptane, and isopropyl ether.

In a second aspect, the present disclosure further provides a pharmaceutically acceptable salt of the compound represented by formula I, wherein the pharmaceutically acceptable salt is selected from the group consisting of succinate, mandelate, oxalate, phosphate, hydrochloride, maleate, fumarate, hydrobromide, citrate, sulfate, mesylate, hippurate, benzoate, malate, and acetate, preferably succinate and mandelate, and more preferably succinate and D-mandelate.

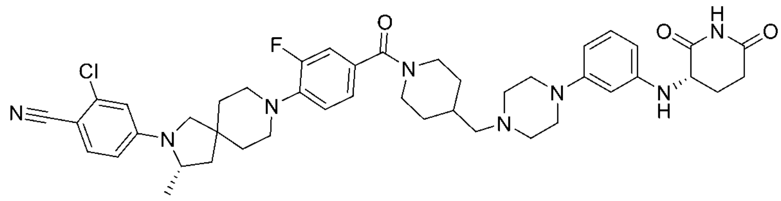

I

In some embodiments, a chemical ratio of the compound represented by formula I to an acid molecule or an acid radical is 2:0.5-1:3; preferably, the chemical ratio of the compound represented by formula I to the acid molecule or the acid radical is selected from the group consisting of 2:1-1:3; more preferably, the chemical ratio of the compound represented by formula I to the acid molecule or the acid radical is selected from the group consisting of 1:0.5, 1:1, 1:1.5, and 1:2. The present disclosure further provides a crystal form A of succinate of the compound represented by formula I, wherein an X-ray powder diffraction pattern expressed in terms of 2$\theta$ angles, which are diffraction angles, of the crystal form has

characteristic peaks at 6.170, 11.215, 12.380, 15.558, 18.230, and 18.884.

In some embodiments, the X-ray powder diffraction pattern expressed in terms of 2$\theta$ angles, which are diffraction angles, of the crystal form A of succinate of the compound represented by formula I has characteristic peaks at 6.170, 9.506, 11.215, 12.380, 15.558, 18.230, 18.884, 19.429, 21.392, and 23.609.

In some embodiments, the X-ray powder diffraction pattern expressed in terms of 2$\theta$ angles, which are diffraction angles, of the crystal form A of succinate of the compound represented by formula I is shown in FIG. 2.

In some embodiments, in the crystal form A of succinate of the compound represented by formula I, a chemical ratio of the compound of formula I to succinate ion or succinic acid is about 1:1-1:2, preferably 1:1 or 1:2.

In some embodiments, a DSC thermogram of the crystal form A of succinate of the compound represented by formula I shows an endothermic peak at 97.22$\pm$3 °C.

The present disclosure further provides a crystal form B of succinate of the compound represented by formula I, wherein an X-ray powder diffraction pattern expressed in terms of 2$\theta$ angles, which are diffraction angles, of the crystal form has characteristic peaks at 6.402, 9.400, 15.728, 17.957, and 19.070.

In some embodiments, the crystal form B of succinate of the compound represented by formula I has characteristic peaks at 6.402, 9.400, 15.728, 17.957, 19.070, and 21.589.

In some embodiments, the X-ray powder diffraction pattern expressed in terms of 2$\theta$ angles, which are diffraction angles, of the crystal form B of succinate of the compound represented by formula I is shown in FIG. 3.

In some embodiments, a DSC thermogram of the crystal form B of succinate of the compound represented by formula I shows an endothermic peak at 121.36$\pm$3 °C.

In some embodiments, in the crystal form B of succinate of the compound represented by formula I, a chemical ratio of the compound of formula I to succinate ion or succinic acid is about 1:1-1:2, preferably 1:1 or 1:2.

The present disclosure further provides a crystal form C of succinate of the compound represented by formula I, wherein an X-ray powder diffraction pattern expressed in terms of 2$\theta$ angles, which are diffraction angles, of the crystal form has characteristic peaks at 5.555, 5.588, 9.363, 11.165, 18.229, and 18.668.

In some embodiments, the crystal form C of succinate of the compound represented by formula I has characteristic peaks at 5.555, 5.588, 9.363, 11.165, 15.505, 18.229, 18.668, 19.734, and 21.920. In some embodiments, the crystal form C of succinate of the compound represented by formula I has characteristic peaks at 5.555, 5.588, 9.363, 11.165, 15.505, 18.229, 18.668, 19.734, 21.920, and 26.943.

In some embodiments, the X-ray powder diffraction pattern expressed in terms of 2$\theta$ angles, which are diffraction angles, of the crystal form C of succinate of the compound represented by formula I is shown in FIG. 4.

In some embodiments, a DSC thermogram of the crystal form C of succinate of the compound represented by formula I shows endothermic peaks at 93.71$\pm$3 °C and 118.69$\pm$3 °C.

In some embodiments, in the crystal form C of succinate of the compound represented by formula I, a chemical ratio of the compound of formula I to succinate ion or succinic acid is about 1:1-1:2, preferably 1:1 or 1:2.

The present disclosure provides a preparation method for the crystal form C of succinate described above, which comprises: mixing the compound represented by formula I with tetrahydrofuran and succinic acid, and crystallizing to give the crystal form C of succinate.

The present disclosure further provides a crystal form D of succinate of the compound represented by formula I, wherein an X-ray powder diffraction pattern expressed in terms of 2$\theta$ angles, which are diffraction angles, of the crystal form has characteristic peaks at 6.878, 10.856, 11.784, 18.225, and 18.597.

In some embodiments, the crystal form D of succinate of the compound represented by formula I has characteristic peaks at 6.878, 10.856, 11.784, 15.639, 17.314, 18.225, 18.597, 19.103, 19.755, 20.155, and 21.477.

In some embodiments, the crystal form D of succinate of the compound represented by formula I has characteristic peaks at 6.878, 10.856, 11.784, 15.639, 17.314, 18.225, 18.597, 19.103, 19.755, 20.155, 21.477, and 27.367.

In some embodiments, the crystal form D of succinate of the compound represented by formula I has characteristic peaks at 6.878, 10.856, 11.784, 15.639, 17.314, 18.225, 18.597, 19.103, 19.755, 20.155, 21.477, 22.268, 24.672, 25.655, 27.367, 28.023, and 28.715.

In some embodiments, the X-ray powder diffraction pattern expressed in terms of 2$\theta$ angles, which are diffraction angles, of the crystal form D of succinate of the compound represented by formula I is shown in FIG. 5.

In some embodiments, a DSC thermogram of the crystal form D of succinate of the compound represented by formula I shows an endothermic peak at 132.93$\pm$3 °C.

In some embodiments, in the crystal form D of succinate of the compound represented by formula I, a chemical ratio of the compound of formula I to succinate ion or succinic acid is about 1:1-1:2, preferably 1:1 or 1:2.

The present disclosure provides a preparation method for the crystal form D of succinate described above, which comprises: mixing the compound represented by formula I with acetone and succinic acid, and crystallizing at a low temperature to give the crystal form D of succinate.

The present disclosure provides a crystal form A of D-mandelate of the compound represented by formula I,

wherein an X-ray powder diffraction pattern expressed in terms of 2$\theta$ angles, which are diffraction angles, of the crystal form has characteristic peaks at 10.554, 13.168, 15.728, 17.944, and 19.784.

In some embodiments, the crystal form A of D-mandelate of the compound represented by formula I has characteristic peaks at 6.159, 8.371, 10.554, 13.168, 15.728, 17.944, 19.784, and 20.381.

In some embodiments, the crystal form A of D-mandelate of the compound represented by formula I has characteristic peaks at 6.159, 8.371, 10.554, 13.168, 15.728, 17.944, 19.784, 20.381, 21.121, and 21.434.

Further preferably, the X-ray powder diffraction pattern expressed in terms of 2$\theta$ angles, which are diffraction angles, of the crystal form is shown in FIG. 6.

In some embodiments, a DSC thermogram of the crystal form A of D-mandelate of the compound represented by formula I shows an endothermic peak at 176.10$\pm$3 °C.

In some embodiments, in the crystal form A of D-mandelate of the compound represented by formula I, a chemical ratio of the compound of formula I to mandelate ion or mandelic acid is about 1:1.

The present disclosure further provides a preparation method for the crystal form A of D-mandelate of the compound represented by formula I, which comprises: mixing the compound represented by formula I with a solvent and D-mandelic acid, and crystallizing to give the crystal form A of D-mandelate, wherein the solvent is selected from the group consisting of one or more of isopropanol, ethanol, and ethyl acetate, and preferably, the solvent is selected from the group consisting of isopropanol/ethyl acetate, ethanol/ethyl acetate, and ethyl acetate.

The present disclosure provides a crystal form I of oxalate of the compound represented by formula I, wherein an X-ray powder diffraction pattern expressed in terms of 2$\theta$ angles, which are diffraction angles, of the crystal form has characteristic peaks at 4.885, 11.219, 12.092, 14.565, 16.383, and 18.201.

Further preferably, the X-ray powder diffraction pattern expressed in terms of 2$\theta$ angles, which are diffraction angles, of the crystal form is shown in FIG. 7.

In some embodiments, a DSC thermogram of the crystal form I of oxalate of the compound represented by formula I shows an endothermic peak at 136.67$\pm$3 °C.

In some embodiments, in the crystal form I of oxalate of the compound represented by formula I, a chemical ratio of the compound of formula I to oxalate ion or oxalic acid is about 1:1-1:3, preferably 1:1 or 1:2.

The present disclosure further provides a preparation method for the crystal form I of oxalate of the compound represented by formula I, which comprises: mixing the compound represented by formula I with acetonitrile and oxalic acid, and crystallizing to give the crystal form I of oxalate. The present disclosure provides a crystal form I of hydrochloride of the compound represented by formula I, wherein an X-ray powder diffraction pattern expressed in terms of 2$\theta$ angles, which are diffraction angles, of the crystal form has characteristic peaks at 4.891, 11.255, 16.565, 17.947, and 22.056.

Further preferably, the X-ray powder diffraction pattern expressed in terms of 2$\theta$ angles, which are diffraction angles, of the crystal form is shown in FIG. 9.

In some embodiments, a DSC thermogram of the crystal form I of hydrochloride of the compound represented by formula I shows an endothermic peak at 194.65$\pm$3 °C.

In some embodiments, in the crystal form I of hydrochloride of the compound represented by formula I, a chemical ratio of the compound of formula I to chloride ion or hydrogen chloride is about 2:1-1:3, preferably 1:1 or 1:2.

The present disclosure further provides a preparation method for hydrochloric acid of the compound represented by formula I, which comprises: mixing the compound represented by formula I with acetonitrile and hydrochloric acid, and crystallizing to give the crystal form I of hydrochloride.

The present disclosure further provides an amorphous form of phosphate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 8.

The present disclosure further provides an amorphous form of maleate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 10.

The present disclosure further provides an amorphous form of fumarate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 11.

The present disclosure further provides an amorphous form of hydrobromide of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 12.

The present disclosure further provides an amorphous form of citrate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 13.

The present disclosure further provides an amorphous form of sulfate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 14.

The present disclosure further provides an amorphous form of mesylate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 15.

The present disclosure further provides an amorphous form of hippurate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 16.

The present disclosure further provides an amorphous form of benzoate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 17.

The present disclosure further provides an amorphous form of malate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 18.

The present disclosure further provides an amorphous form of acetate of the compound represented by formula I, wherein an X-ray powder diffraction pattern of the amorphous form has no sharp peaks and preferably has non-sharp peaks; more preferably, the X-ray powder diffraction pattern of the amorphous form is shown in FIG. 19.

The method for the crystallization described in the present disclosure is conventional, for example, volatilizing crystallization, cooling crystallization, slurrying crystallization, room-temperature crystallization, and stirring crystallization.

The present disclosure further provides a pharmaceutical composition, which comprises: the amorphous form, the pharmaceutically acceptable salt, or the crystal form described above, or the crystal form prepared by the preparation method described above; and optionally a pharmaceutically acceptable excipient.

The present disclosure further provides a preparation method for the pharmaceutical composition, which comprises a step of mixing the amorphous form, the pharmaceutically acceptable salt, or the crystal form described above, or the crystal form prepared by the preparation method described above with the pharmaceutically acceptable excipient.

The present disclosure further provides use of the amorphous form, the pharmaceutically acceptable salt, or the crystal form described above, the crystal form prepared by the preparation method described above, or the pharmaceutical composition described above in the manufacture of a medicament for regulating the ubiquitination and degradation of the androgen receptor (AR) protein.

The present disclosure further provides use of the amorphous form, the pharmaceutically acceptable salt, or the crystal form described above, the crystal form prepared by the preparation method described above, or the pharmaceutical composition described above in regulating the ubiquitination and degradation of the androgen receptor (AR) protein.

The present disclosure further provides the amorphous form, the pharmaceutically acceptable salt, or the crystal form described above, the crystal form prepared by the preparation method described above, or the pharmaceutical composition described above for use in regulating the ubiquitination and degradation of the androgen receptor (AR) protein.

The present disclosure further provides use of the amorphous form, the pharmaceutically acceptable salt, or the crystal form described above, the crystal form prepared by the preparation method described above, or the pharmaceutical composition described above in the manufacture of a medicament for treating and/or preventing an androgen receptor-mediated or -dependent disease or disorder, wherein the androgen receptor-mediated or -dependent disease or disorder is preferably selected from the group consisting of a tumor, male sexual dysfunction, and Kennedy's disease.

The present disclosure further provides use of the amorphous form, the pharmaceutically acceptable salt, or the crystal form described above, the crystal form prepared by the preparation method described above, or the pharmaceutical composition described above in treating and/or preventing an androgen receptor-mediated or -dependent disease or disorder, wherein the androgen receptor-mediated or -dependent disease or disorder is preferably selected from the group consisting of a tumor, male sexual dysfunction, and Kennedy's disease.

The present disclosure further provides the amorphous form, the pharmaceutically acceptable salt, or the crystal form described above, the crystal form prepared by the preparation method described above, or the pharmaceutical composition described above for use in treating and/or preventing an androgen receptor-mediated or -dependent disease or disorder, wherein the androgen receptor-mediated or -dependent disease or disorder is preferably selected from the group consisting of a tumor, male sexual dysfunction, and Kennedy's disease.

In some embodiments, the androgen receptor-mediated or -dependent disease or disorder is selected from the group consisting of prostate cancer, prostatic hyperplasia, hirsutism, alopecia, anorexia nervosa, breast cancer, acne, male sexual dysfunction, Kennedy's disease, and AIDS, preferably prostate cancer, and more preferably hormone-sensitive prostate cancer or hormone-refractory prostate cancer.

As used herein, "$2\theta$ or $2\theta$ angle" refers to a diffraction angle; $\theta$ refers to the Bragg angle in ° or degrees; the 20 of each characteristic peak has a margin of error of $\pm 0.2$ (including the case that a number having more than 1 decimal place

is rounded), and the margin of error may be -0.20, -0.19, -0.18, -0.17, -0.16, -0.15, -0.14, -0.13, -0.12, -0.11, -0.10, -0.09, -0.08, -0.07, -0.06, -0.05, -0.04, - 0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

According to the description of hygroscopicity characteristics and the definitions of hygroscopic weight gains in "General Rule 9103, Guidelines for Hygroscopicity Trials for Pharmaceuticals" in the Chinese Pharmacopoeia, Volume IV, 2015 Edition,

deliquescent: sufficient water is absorbed to form a liquid;
extremely hygroscopic: the hygroscopic weight gain is not less than 15%;
hygroscopic: the hygroscopic weight gain is less than 15% but not less than 2%;
slightly hygroscopic: the hygroscopic weight gain is less than 2% but not less than 0.2%;
nonhygroscopic or practically nonhygroscopic: the hygroscopic weight gain is less than 0.2%.

As used herein, the "differential scanning calorimetry" or "DSC" refers to the measurement of the temperature difference and heat flow difference between a sample and a reference substance during a ramping or thermostatic process to characterize all the physical changes and chemical changes related to the thermal effect, and to acquire phase change information about the sample.

As used herein, the drying temperature is generally 25 °C-100 °C, preferably 40 °C-70 °C, and the drying may be performed under atmospheric pressure or a reduced pressure of < -0.08 MPa.

As used herein, the "excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

As used herein, "slurrying" or "slurried" refers to a purification method utilizing the low solubility of the target substance and high solubility of the impurities in a solvent, which is capable of decoloring, changing the crystal form, or removing small amounts of impurities.

The starting materials used in the preparation methods for the crystal forms of the present disclosure may be any form of the compound; specific forms include, but are not limited to: an amorphous form, any crystal form, a hydrate, a solvate, etc.

In the present disclosure, numerical values such as the content of related substances are data obtained by measurement and calculation, and there is inevitably a certain degree of error. In general, ±10% is a reasonable margin of error. There is a certain degree of error change depending on the context where it is used, and the error change is no more than ±10% and may be ±9%, ±8%, ±7%, ±6%, ±5%, ±4%, ±3%, ±2%, or ±1%, preferably ±5%.

The compound of formula I of the present disclosure, 2-chloro-4-((S)-8-(4-(4-((4-(3-(((S)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile (hereinafter referred to as compound I), was prepared with reference to the method described in PCT/CN2022/134333, and relevant contents are incorporated herein by reference for illustrative purposes.

## BRIEF DESCRIPTION OF THE DRAWINGS

FIG. 1 shows an XPRD pattern of the amorphous form of formula I.
FIG. 2 shows an XPRD pattern of the crystal form A of succinate of formula I.
FIG. 3 shows an XPRD pattern of the crystal form B of succinate of formula I.
FIG. 4 shows an XPRD pattern of the crystal form C of succinate of formula I.
FIG. 5 shows an XPRD pattern of the crystal form D of succinate of formula I.
FIG. 6 shows an XPRD pattern of the crystal form A of mandelate of formula I.
FIG. 7 shows an XPRD pattern of the crystal form I of oxalate of formula I.
FIG. 8 shows an XPRD pattern of the amorphous form of phosphate of formula I.
FIG. 9 shows an XPRD pattern of the crystal form I of hydrochloride of formula I.
FIG. 10 shows an XPRD pattern of the amorphous form of maleate of formula I.
FIG. 11 shows an XPRD pattern of the amorphous form of fumarate of formula I.
FIG. 12 shows an XPRD pattern of the amorphous form of hydrobromide of formula I.
FIG. 13 shows an XPRD pattern of the amorphous form of citrate of formula I.
FIG. 14 shows an XPRD pattern of the amorphous form of citrate of formula I.
FIG. 15 shows an XPRD pattern of the amorphous form of mesylate of formula I.
FIG. 16 shows an XPRD pattern of the amorphous form of hippurate of formula I.

FIG. 17 shows an XPRD pattern of the amorphous form of benzoate of formula I.
FIG. 18 shows an XPRD pattern of the amorphous form of malate of formula I.
FIG. 19 shows an XPRD pattern of the amorphous form of acetate of formula I.

## DETAILED DESCRIPTION

The present disclosure is further described below with reference to examples, but these examples are not intended to limit the scope of the present disclosure.

Examples

The structures of the compounds were determined by nuclear magnetic resonance (NMR) spectroscopy or/and mass spectrometry (MS). The NMR shifts (δ) are given in $10^{-6}$ (ppm). The NMR analyses were performed using a Bruker AVANCE-400 nuclear magnetic resonance instrument or Bruker AVANCE NEO 500M, with dimethyl sulfoxide-D6 (DMSO-$d_6$), chloroform-D ($CDCl_3$), and methanol-D4 ($CD_3OD$) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS),

waters ACQuity UPLC-QD/SQD (manufacturer: waters; MS model: waters ACQuity Qda Detector/waters SQ Detector), and
THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high performance liquid chromatographs.

The chiral HPLC analyses were performed using an Agilent 1260 DAD high performance liquid chromatograph.

The preparative high performance liquid chromatography was performed using Waters 2545-2767, Waters 2767-SQ Detecor2, Shimadzu LC-20AP, and Gilson GX-281 preparative chromatographs. The preparative chiral chromatography was performed using a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

The silica gel column chromatography generally used Yantai Huanghai 200-300 mesh silica gel as the carrier.

The mean kinase inhibition rates and $IC_{50}$ values were measured using a NovoStar microplate reader (BMG, Germany).

The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed in an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenation apparatus and a Qinglan QL-500 hydrogen generator, or an HC2-SS hydrogenation apparatus. The hydrogenation reactions generally involved 3 cycles of vacuumization and hydrogen filling. The microwave reactions were performed using a CEM Discover-S 908860 microwave reactor. In the examples, the solutions were aqueous solutions unless otherwise specified.

In the examples, the reaction temperature was room temperature, i.e., 20 °C-30 °C, unless otherwise specified.

The monitoring of the reaction progress in the examples was performed using thin-layer chromatography (TLC). The developing solvents used in the reactions, the eluent systems used in the column chromatography purification of compounds, and the developing solvent systems used in the thin-layer chromatography include: A: a dichloromethane/methanol system, and B: a n-hexane/ethyl acetate system. The volume ratio of the solvents was adjusted according to the polarity of the compound, or by adding a small amount of a basic or acidic reagent such as triethylamine and acetic acid.

XRPD refers to X-ray powder diffraction: The analysis was performed using a BRUKER D8 X-ray diffractometer; specific acquisition information: a Cu anode (40 kV, 40 mA), Cu-Kα1 ray (λ = 1.54060 Å), Kα2 ray (λ = 1.54439 Å), and Kβ ray (λ = 1.39222 Å). Scan range (2θ range): 3-45°. DSC refers to differential scanning calorimetry: The analysis was performed using a METTLER TOLEDO DSC 3+ differential scanning calorimeter, with a temperature ramp rate of 10

°C/min, specific temperature ranges shown in corresponding thermograms (mostly 25-300 °C or 25-350 °C), and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding profiles (mostly 30-350 °C), and a nitrogen purge speed of 50 mL/min.

DVS refers to dynamic vapor sorption: The analysis was performed using SMS DVS Advantage; at 25 °C, the humidity was changed as follows: 50%-95%-0%-95%-50%, in steps of 10% (5% for the last step) (specific humidity ranges are shown in corresponding patterns, and those listed here were used in most cases); the criteria were Tmax ≤ 360 min and dm/dt ≤ 0.002%.

Ion chromatography analysis: instrument: DIONEX AQUION ion chromatograph (USA); detection mode: conductance; separation column: IonPac AS 14A-AG14; eluent: $NaHCO_3$ 0.5 mM + $Na_2CO_3$ 4.0 mM; flow rate: 1.0 mL/min.

**Example 1:** The compound of formula I was synthesized using the preparation method described in Example **15-1** of Patent Application No. PCT/CN2022/134333.

2-Chloro-4-((S)-8-(4-(4-((4-(3-(((S)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile **1-1** (formula I)

2-Chloro-4-((S)-8-(4-(4-((4-(3-(((R)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile 1-2

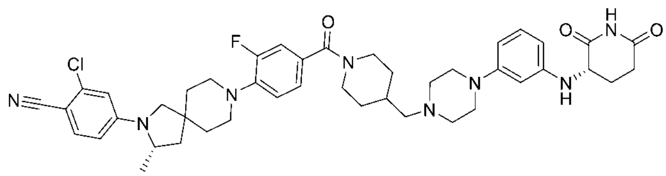

**1-1**

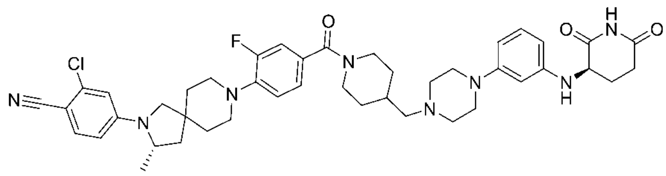

**1-2**

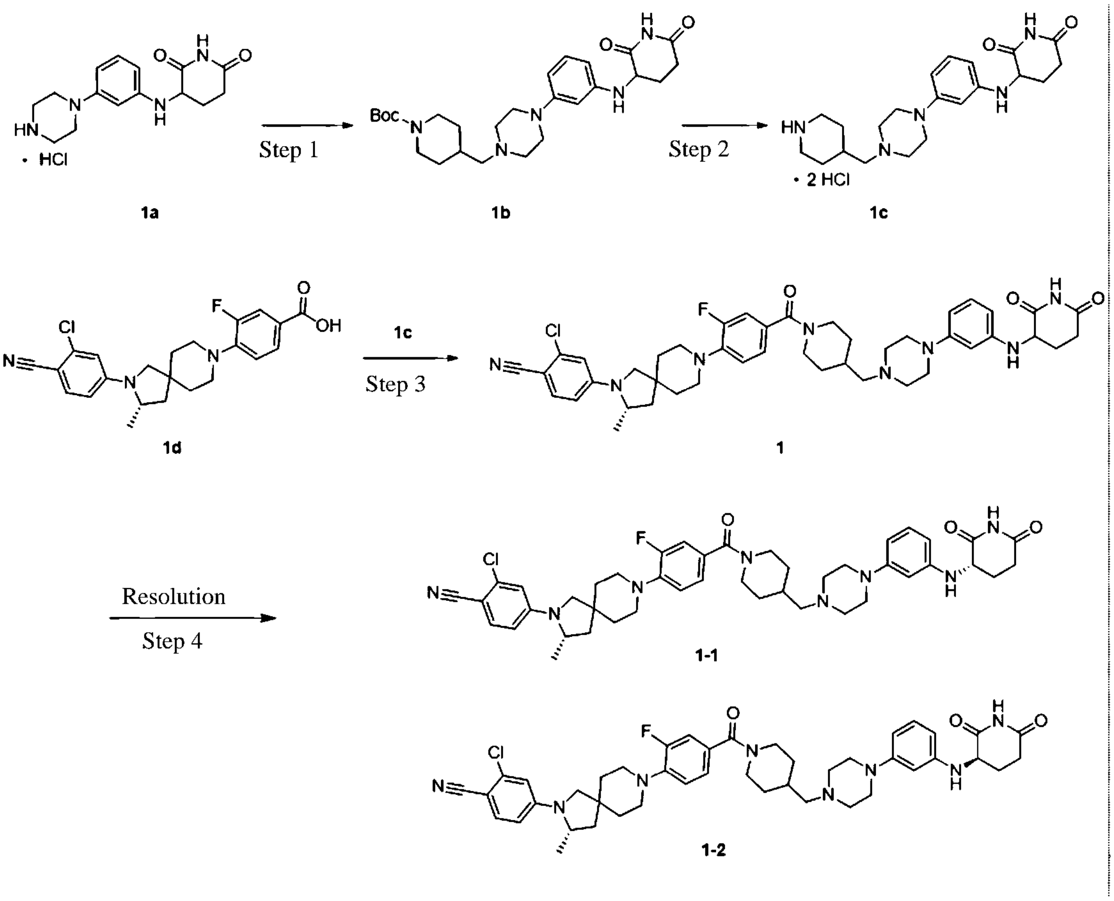


Step 1

tert-Butyl (±)-4-((4-(3-((2,6-dioxopiperidin-3-yl)amino)phenyl)piperazin-1-yl)methyl)piperidine-1-carboxylate 1b

To a 25 mL three-necked flask were added 3 mL of a mixed solution of dichloromethane and methanol (V/V = 2/1), followed by (±)-3-((3-(piperazin-1-yl)phenyl)amino)piperidine-2,6-dione **1a** (90 mg, 0.25 mmol, prepared using the method disclosed for "Compound 86 on page 266 of the specification in Patent Application No. WO 2018237026 A1") and anhydrous sodium acetate (102 mg, 1.24 mmol). After 15 min of reaction, tert-butyl 4-formylpiperidine-1-carboxylate (85 mg, 0.40 mmol) was added. After another 30 min of reaction, sodium triacetoxyborohydride (106 mg, 0.50 mmol) was slowly added, and then the mixture was left to react for 16 h. 10 mL of a saturated sodium bicarbonate solution was added to the system under ice-bath cooling to quench the reaction, and extraction was performed with dichloromethane (20 mL × 3). The organic phases were combined, washed with a saturated sodium chloride solution (20 mL × 3), dried over anhydrous sodium sulfate, and filtered. The filtrate was concentrated under reduced pressure, and the resulting residue was purified by silica gel column chromatography with eluent system B to give the title compound **1b** (110 mg, yield: 90%).

MS m/z (ESI): 486.6 [M+1].

Step 2

(±)-3-((3-(4-(Piperidin-4-ylmethyl)piperazin-1-yl)phenyl)amino)piperidine-2,6-dione dihydrochloride 1c

Compound **1b** (105 mg, 0.22 mmol) was dissolved in 3 mL of dichloromethane, and a 4 M solution of hydrogen chloride in 1,4-dioxane (0.53 mL) was slowly added in an ice bath. The mixture was left to react for 4 h. The reaction mixture was concentrated, and the residue was dried *in vacuo* to give a crude product of the title compound **1c** (99 mg, yield: 98%). The crude product was directly used in the next step without purification.

MS m/z (ESI): 386.5 [M+1].

Step 3

2-Chloro-4-((3S)-8-(4-(4-((4-(3-((2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile 1 (a mixture of diastereomers)

To a 25 mL three-necked flask were sequentially added 2 mL of N,N-dimethylformamide, compound 1c (50 mg, 0.094 mmol), and (S)-4-(2-(3-chloro-4-cyanophenyl)-3-methyl-2,8-diazaspiro[4.5]decan-8-yl)-3-fluorobenzoic acid 1d (45 mg, 0.083 mmol, prepared using the method disclosed in "Example 52 on page 271 of the specification in Patent Application No. WO2021055756A1"). N,N-Diisopropylethylamine (85 mg, 0.66 mmol) and O-(7-azabenzotriazol-1-yl)-N,N,N',N'-tetramethyluronium hexafluorophosphate (63 mg, 0.16 mmol) were slowly added in an ice bath. The cooling bath was removed, and then the mixture was left to react for 16 h. The reaction mixture was filtered and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound 1 (a 1:1 mixture of diastereomers, 36 mg, yield: 54%).

MS m/z (ESI): 795.3 [M+1];
$^1$H NMR (500 MHz, DMSO-$d_6$): δ 10.75 (s, 1H), 7.59 (d, 1H), 7.13 (d, 1H), 7.11 (s, 1H), 7.06 (t, 1H), 6.89 (t, 1H), 6.79 (d, 1H), 6.65 (dd, 1H), 6.24 (s, 1H), 6.17 (d, 1H), 6.12 (d, 1H), 5.60 (d, 1H), 4.35-4.25 (m, 1H), 4.07-3.97 (m, 1H), 3.43 (d, 1H), 3.34-3.30 (m, 3H), 3.17-2.92(m, 8H), 2.78-2.68 (m, 1H), 2.64-2.50 (m, 3H), 2.48-2.40 (m, 4H), 2.28-1.93 (m, 4H), 1.90-1.67 (m, 5H), 1.62-1.48 (m, 3H), 1.27-1.15 (m, 4H), 1.12-0.99 (m, 2H).

Step 4

2-Chloro-4-((S)-8-(4-(4-((4-(3-(((S)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile 1-1 (formula I)

2-Chloro-4-((S)-8-(4-(4-((4-(3-(((R)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile **1-2**

Compound **1** (21 mg) was resolved by preparative chiral chromatography (separation conditions: Gilson-281, chromatographic column: Phenomenex Amylose-2, 5 μm, 21.2 mm × 250 mm, mobile phase: acetonitrile (0.5% 7 M ammonia ($NH_3$) in methanol)/ethanol (0.5% 7 M ammonia ($NH_3$) in methanol) = 40/60 (v/v)), flow rate 20 mL/min) to give the title compound **1-2** (5 mg) and compound **1-1** (8 mg).
**1-2** (5 mg, yield: 24%), with a shorter retention time:

MS m/z (ESI): 795.3 [M+1].
Chiral HPLC analysis method: retention time: 3.53 min (Agilent1260 DAD, chromatographic column: Phenomenex LUX Amylose-2, (4.6 × 150 mM), 5 μm; mobile phase: acetonitrile/ethanol/diethylamine = 40/60/0.06 (v/v/v), flow rate 1 mL/min);
$^1$H NMR (500 MHz, DMSO-$d_6$): δ 10.75 (s, 1H), 7.59 (d, 1H), 7.14 (d, 1H), 7.12 (s, 1H), 7.06 (t, 1H), 6.90 (t, 1H), 6.79 (d, 1H), 6.65 (dd, 1H), 6.24 (s, 1H), 6.17 (d, 1H), 6.13 (d, 1H), 5.61 (d, 1H), 4.35-4.25 (m, 1H), 4.07-3.97 (m, 1H), 3.43 (d, 1H), 3.37-3.30 (m, 3H), 3.17-2.92 (m, 8H), 2.78-2.68 (m, 1H), 2.64-2.50 (m, 3H), 2.48-2.40 (m, 4H), 2.28-1.93 (m, 4H), 1.90-1.67 (m, 5H), 1.62-1.48 (m, 3H), 1.27-1.15 (m, 4H), 1.12-0.99 (m, 2H).

Compound **1-1** (8 mg, yield: 38%), with a longer retention time:

MS m/z (ESI): 795.3 [M+1].
Chiral HPLC analysis method: retention time: 4.19 min (Agilent1260 DAD, chromatographic column: Phenomenex LUX Amylose-2, (4.6 × 150 mM), 5 μm; mobile phase: acetonitrile/ethanol/diethylamine = 40/60/0.06 (v/v/v), flow rate 1 mL/min);
$^1$H NMR (500 MHz, DMSO-$d_6$): δ 10.76 (s, 1H), 7.59 (d, 1H), 7.13 (d, 1H), 7.11 (s, 1H), 7.06 (t, 1H), 6.90 (t, 1H), 6.80 (d, 1H), 6.65 (dd, 1H), 6.24 (s, 1H), 6.17 (d, 1H), 6.12 (d, 1H), 5.61 (d, 1H), 4.35-4.25 (m, 1H), 4.07-3.97 (m, 1H), 3.43 (d, 1H), 3.34-3.30 (m, 3H), 3.17-2.92(m, 8H), 2.78-2.68 (m, 1H), 2.64-2.50 (m, 3H), 2.48-2.40 (m, 4H), 2.28-1.93 (m, 4H), 1.90-1.67 (m, 5H), 1.62-1.48 (m, 3H), 1.27-1.15 (m, 4H), 1.12-0.99 (m, 2H).

Biological Evaluations

The present disclosure is further described and explained below with reference to test examples. However, these test examples are not intended to limit the scope of the present disclosure.

Test Example 1. Determination of Degradation Activity of Compounds of the Present Disclosure on Androgen Receptor in LNCaP Cells

Through the *in vitro* cell assay described below, the degradation activity of the test compounds on the androgen receptor (AR) in the human prostate cancer cell strain can be determined, and their activity can be represented by $DC_{50}$ values. On the first day of the experiment, LNCaP cells (ATCC, CRL-1740) were seeded in a 96-well plate at a density of 20,000 cells/well using phenol red-free RPMI 1640 medium (Gibco, 11835-030) containing 5% charcoal-stripped fetal bovine serum (Shanghai Biosun Biotechnology Co., Ltd., S-FBS-AU-045), with 200 μL of cell suspension in each well. The plate was incubated in a 5% $CO_2$ cell incubator at 37 °C for three days. On the fourth day, serially diluted test compounds prepared with a plating medium containing 1 nM R1881 (Aladdin, M305037) were added at 22 μL/well. The final concentration of R1881 was 0.1 nM, and the final concentrations of the compounds were 7 concentration points obtained by 6-fold serial dilution starting from 2.5 μM. Blank control cell wells containing 0.5% DMSO were set up. The plate was incubated in a 5% $CO_2$ cell incubator at 37 °C for 24 h. On the fifth day, the 96-well cell culture plate was taken out, and the cell culture supernatant was removed from the plate using a pipette and discarded. The plate was washed once with 300 μL of ice-cold PBS, and 50 μL of 1× lysis buffer containing 1 mM PMSF diluted with $ddH_2O$ (Cell Signaling Technology, #9803S) was then added. After 1 min of shaking on a micro-shaker, the cell plate was placed on ice for 15-20 min of lysis. The lysate was well mixed by pipetting and then centrifuged at 4000 rpm and 4 °C for 10 min. AR protein levels were measured using an AR ELISA kit (Cell Signaling Technology, #12850) according to the instructions. 96 μL of sample dilution and 4 μL of sample lysate or blank lysis buffer were added to a 96-well microplate. The plate was sealed, shaken to mix well the contents, and then incubated in an incubator at 37 °C for 2 h. The liquid in the wells was discarded, and 300 μL of wash buffer was added to each well to wash the plate. After five washes, 100 μL of detection antibody working solution was added. The plate was sealed and incubated in an incubator at 37 °C for 1 h. The liquid in the wells was discarded, and 300 μL of wash buffer was added to each well to wash the plate. After five washes, 100 μL of enzyme conjugate working solution was added. The plate was sealed and then incubated in an incubator at 37 °C for 30 min. The liquid in the wells was discarded, and 300 μL of wash buffer was added to each well to wash the plate. After five washes, 100 μL of substrate solution was added. The plate was sealed and then incubated at room temperature in the dark for 5 min, and 100 μL of stop solution was added to each well. Immediately after the contents were well mixed, the plate was placed on a microplate reader (BMG Labtech, PHERAstar FS), and the OD450 and OD540 values were measured. The OD540 absorbance values of the corresponding wells were subtracted from the OD450 absorbance values of all the wells to obtain corrected OD450 absorbance values (OD450-correction). The degradation rate of each concentration of the compounds was calculated using the following formula. The maximums (Max) represent the maximum AR degradation rates of the compounds. Using GraphPad Prism, curve fitting was performed based on the logarithmic concentration and degradation efficiency of each of the compounds, and the $DC_{50}$ value was calculated.

Degradation rate (%) = $(\text{OD450-correction}_{\text{blank control}} - \text{OD450-correction}_{\text{compound}})/(\text{OD450- correction}_{\text{blank control}} - \text{OD450-correction}_{\text{lysis buffer control}}) \times 100\%$

The biological activity of the compounds of the present disclosure was obtained from the above analysis. The calculated $DC_{50}$ and Max values are shown in Table 1 below.

Table 1. The degradation activity of the compounds of the present disclosure on the androgen receptor in LNCaP cells

| Example No. | LNCaP $DC_{50}$ (nM) | Max (%) |
|---|---|---|
| 1 | 2.5 | 96 |
| 1-2 | 14.2 | 97 |
| 1-1 | 4.2 | 97 |

Conclusion: The compounds of the present disclosure exhibited significant degradation activity on the androgen receptor in LNCaP cells.

Test Example 2. Determination of Inhibition of LNCaP Proliferation by Compounds of the Present Disclosure

Through the *in vitro* cell assay described below, the inhibitory activity of the test compounds against the proliferation of the human prostate cancer cell strain can be determined, and their activity can be represented by $IC_{50}$ values. On the first day of the experiment, LNCaP cells (ATCC, CRL-1740) were seeded in a 96-well plate at a density of 7500 cells/well using phenol red-free RPMI 1640 medium (Gibco, 11835-030) containing 5% charcoal-stripped fetal

bovine serum (Shanghai Biosun Biotechnology Co., Ltd., S-FBS-AU-045), with 200 μL of cell suspension in each well. The plate was incubated in a 5% $CO_2$ cell incubator at 37 °C for three days. On the fourth day, serially diluted test compounds prepared with a plating medium containing 1 nM R1881 (Aladdin, M305037) were added at 22 μL/well. The final concentration of R1881 was 0.1 nM, and the final concentrations of the compounds were 9 concentration points obtained by 4-fold serial dilution starting from 10 μM. Blank control cell wells containing 0.5% DMSO and cell-free vehicle control wells were set up. The plate was incubated in a 5% $CO_2$ cell incubator at 37 °C for six days. On the tenth day, the 96-well cell culture plate was taken out, and 110 μL of cell culture supernatant was removed from each well using a pipette and discarded. Then, 50 μL of CellTiter-Glo® Luminescent Cell Viability Assay (Promega, G7573) was added. The plate was left to stand at room temperature for 10 min, and then luminescence signal values (RLU) were obtained using a multifunctional microplate reader (PerkinElmer, VICTOR 3). The inhibition rate of each concentration of the compounds was calculated using the following formula. The maximums (Max) represent the maximum proliferation inhibition rates of the compounds. Using GraphPad Prism, curve fitting was performed based on the logarithmic concentration and inhibition rate of each of the compounds, and the $IC_{50}$ value was calculated.

$$\text{Inhibition rate (\%)} = (RLU_{\text{blank control}} - RLU_{\text{compound}})/(RLU_{\text{blank control}} - RLU_{\text{vehicle control}}) \times 100\%$$

The biological activity of the compounds of the present disclosure was obtained from the above analysis. The calculated $IC_{50}$ values are shown in Table 2 below.

Table 2. The inhibitory activity of the compounds of the present disclosure against the proliferation of LNCaP cells

| Compound No. | LNCaP $IC_{50}$ (nM) | Max (%) |
|---|---|---|
| 1 | 65 | 74 |
| 1-2 | 74 | 68 |
| 1-1 | 15 | 64 |

Conclusion: The compounds of the present disclosure exhibited good inhibitory activity against the proliferation of LNCaP cells.

Test Example 3. Pharmacokinetic Evaluation

Comparative Example 1 (synthesized with reference to Example 307 in Patent No. WO2021055756A1) has the following structure:

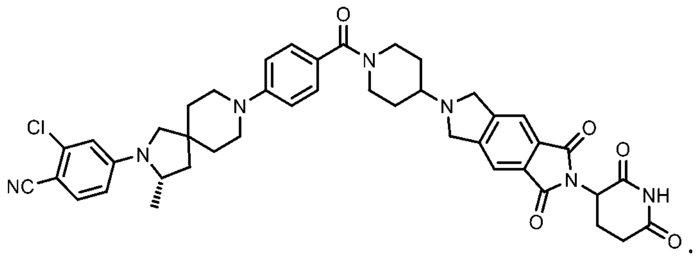

ARV-110 (synthesized with reference to Example 406 in Patent No. WO2018071606A1) has the following structure:

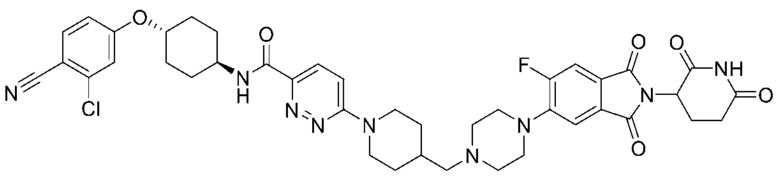

I. Test in SD rats

1. Abstract

SD rats were used as test animals. The plasma concentrations of compounds of the present disclosure in SD rats at different time points after intragastric (i.g.) administration were determined using an LC/MS/MS method. The pharmacokinetic behaviors of the compounds of the present disclosure in SD rats were studied, and their pharmacokinetic profiles were evaluated.

2. Test protocol

2.1. Test compounds

The compound of Example 1;

the compound of Example 1-1; and
the compound of Comparative Example 1.

2.2. Test animals

Twelve SD rats (half male and half female, provided by Vital River Laboratory Animal Technology Co., Ltd.) were evenly divided into 3 groups. The animals were fasted overnight and then subjected to intragastric administration.

2.3. Compound solution preparation

A certain amount of test compound was weighed out and dissolved in 5% DMSO + 5% tween 80 + 90% normal saline to prepare a 0.2 mg/mL clear solution.

2.4. Administration

The administration dose was 2.0 mg/kg, and the administration volume was 10.0 mL/kg.

3. Procedures

0.2-mL blood samples were collected from the orbit before administration and at 0.25 h, 0.5 h, 1.0 h, 2.0 h, 4.0 h, 6.0 h, 8.0 h, 11.0 h, and 24.0 h after administration. The samples were then placed into EDTA-K2 anticoagulation test tubes and centrifuged at 10,000 rpm for 1 min (4 °C), and plasma was separated within 1 h and stored at -20 °C before analysis. The procedures from the blood collection to the centrifugation were performed under ice-bath conditions. Access to food was given 2 h after administration.

After administration of different concentrations of the test compounds, the plasma concentrations of the test compounds in the SD rats were determined as follows: 25 μL of each of the SD rat plasma samples collected at various time points after administration was taken; 200 μL of acetonitrile was added to precipitate protein, and 50 μL of camptothecin (100 ng/mL) was added; the mixture was vortex-mixed for 5 min and then centrifuged at 4000 rpm for 10 min; 30 μL of the supernatant was then taken and diluted with 120 μL of water, and 1 μL of the dilution was taken for LC/MS/MS analysis.

4. Pharmacokinetic parameters

Table 3. Pharmacokinetic parameters of the compounds of the present disclosure in SD rats

| Compound No. | Route of administration/ administration dose (mg/kg) | Plasma concentration Cmax (ng/mL) | Area under curve $AUC_{0-t}$ (h*ng/mL) | Half-life T1/2 (h) | Clearance rate CL/F (mL/min/kg) | Apparent distribution volume Vz/F (mL/kg) |
|---|---|---|---|---|---|---|
| 1 | i.g. /2.0 | 227 | 2137 | 6.75 | 14.6 | 8504 |
| 1-1 | i.g. /2.0 | 110 | 1126 | 6.66 | 39.5 | 23003 |

(continued)

| Compound No. | Route of administration/ administration dose (mg/kg) | Plasma concentration Cmax (ng/mL) | Area under curve $AUC_{0-t}$ (h*ng/mL) | Half-life T1/2 (h) | Clearance rate CL/F (mL/min/kg) | Apparent distribution volume Vz/F (mL/kg) |
|---|---|---|---|---|---|---|
| Comparative Example 1 | i.g. /2.0 | 2.98 | 22 | 4.9 | 1166 | 506710 |

Conclusion: Compared with Comparative Example 1, the compounds of the present disclosure exhibited larger areas under the curves, longer half-lives, and lower clearance rates in SD rats, indicating excellent pharmacokinetic absorption activity and significant pharmacokinetic advantages.

II. Test in dogs

1. Abstract

Beagles were used as test animals. The plasma concentrations of a compound of the present disclosure in beagles at different time points after intragastric (i.g.) administration were determined using an LC/MS/MS method. The pharmacokinetic behavior of the compound of the present disclosure in beagles was studied, and its pharmacokinetic profile was evaluated.

2. Test protocol

2.1. Test compounds

The compound of Example 1-1; and
compound ARV-110.

2.2. Test animals

Eight beagles (half male and half female, provided by Jiangsu Johnbio Biotechnology Co., Ltd.) were evenly divided into 2 groups. The animals were fasted overnight and then subjected to intragastric administration.

2.3. Compound solution preparation

A certain amount of test compound was weighed out and dissolved in 5% DMSO + 30% PG + 30% PEG400 + 35% normal saline to prepare a 0.4 mg/mL solution.

2.4. Administration

The administration dose was 2.0 mg/kg, and the administration volume was 5.0 mL/kg.

3. Procedures

0.5-mL blood samples were collected from the forelimb vein before administration and at 0.25 h, 0.5 h, 1.0 h, 2.0 h, 4.0 h, 6.0 h, 8.0 h, 11.0 h, and 24.0 h after administration. The samples were then placed into EDTA-K2 anticoagulation test tubes and centrifuged at 10,000 rpm for 2 min (4 °C), and plasma was separated within 1 h and stored at -80 °C before analysis. The procedures from the blood collection to the centrifugation were performed under ice-bath conditions. Access to food was given 3 h after administration.

After administration of different concentrations of the test compounds, the plasma concentrations of the test compounds in the beagles were determined as follows: 50 μL of each of the beagle plasma samples collected at various time points after administration was taken; 25 μL of camptothecin (1 μg/mL) and 450 μL of acetonitrile were added to precipitate protein, and the mixture was vortex-mixed and then centrifuged at 3700 rpm for 10 min; 0.5 μL of the supernatant was taken for LC/MS/MS analysis.

4. Pharmacokinetic parameters

Table 4. Pharmacokinetic parameters of the compound of the present disclosure in beagles

| Compound No. | Route of administration/ administration dose (mg/kg) | Plasma concentration Cmax (ng/mL) | Area under curve $AUC_{0-t}$ (h*ng/mL) | Half-life T1/2 (h) | Clearance rate CL/F (mL/min/kg) | Apparent distribution volume Vz/F (mL/kg) |
|---|---|---|---|---|---|---|
| 1-1 | i.g. /2.0 | 75.6 | 1137 | 22.5 | 15.8 | 29843 |
| ARV-110 | i.g. /2.0 | 13.0 | 239 | 48.8 | 70.9 | 278328 |

Conclusion: Compared with compound ARV-110, the compound of the present disclosure exhibited a larger area under the curve and a lower clearance rate in beagles, indicating excellent pharmacokinetic absorption activity and significant pharmacokinetic advantages.

III. Test in monkeys

1. Abstract

Cynomolgus monkeys were used as test animals. The plasma concentrations of a compound of the present disclosure in cynomolgus monkeys at different time points after intragastric (i.g.) administration were determined using an LC/MS/MS method. The pharmacokinetic behavior of the compound of the present disclosure in cynomolgus monkeys was studied, and its pharmacokinetic profile was evaluated.

2. Test protocol

2.1. Test compounds

The compound of Example 1-1; and
ARV-110.

2.2. Test animals

Eight cynomolgus monkeys (half male and half female, provided by Shanghai Medicilon Inc.) were evenly divided into 2 groups. The animals were fasted overnight and then subjected to intragastric administration.

2.3. Compound solution preparation

A certain amount of test compound was weighed out and dissolved in 5% DMSO + 30% PG + 30% PEG400 + 35% normal saline to prepare a 0.4 mg/mL clear solution.

2.4. Administration

The administration dose was 2.0 mg/kg, and the administration volume was 5.0 mL/kg.

3. Procedures

1-mL blood samples were collected from the forelimb vein before administration and at 0.25 h, 0.5 h, 1.0 h, 2.0 h, 4.0 h, 6.0 h, 8.0 h, 12.0 h, and 24.0 h after administration. The samples were then placed into EDTA-K2 anticoagulation test tubes and centrifuged at 2200 g for 10 min (2-8 °C), and plasma was separated within 1 h and stored at -80 °C before analysis. The procedures from the blood collection to the centrifugation were performed under ice-bath conditions. Access to food and *ad libitum* access to water were given 3 h after administration.

After administration of different concentrations of the test compounds, the plasma concentrations of the test compounds in the cynomolgus monkeys were determined as follows: 20 μL of each of the cynomolgus monkey plasma samples collected at various time points after administration was taken; 400 μL of methanol was added to precipitate

protein (containing 10 ng/mL verapamil), and the mixture was vortex-mixed for 1 min and then centrifuged at 18,000 g for 7 min; 5 μL of the supernatant was taken for LC/MS/MS analysis.

4. Pharmacokinetic parameters

Table 5. Pharmacokinetic parameters of the compound of the present disclosure in cynomolgus monkeys

| Compound No. | Route of administration/ administration dose (mg/kg) | Plasma concentration Cmax (ng/mL) | Area under curve $AUC_{0-t}$ (h*ng/mL) | Half-life T1/2 (h) | Clearance rate CL/F (mL/min/kg) | Apparent distribution volume Vz/F (mL/kg) |
|---|---|---|---|---|---|---|
| 1-1 | i.g. /2.0 | 200.5 | 2086.0 | 14.6 | 11.0 | 14494.8 |
| ARV-110 | i.g. /2.0 | 15.9 | 282.0 | 42.0 | 44.6 | 165179.6 |

Conclusion: Compared with ARV-110, the compound of the present disclosure exhibited a larger area under the curve and a lower clearance rate in cynomolgus monkeys, indicating excellent pharmacokinetic absorption activity and significant pharmacokinetic advantages.

**Example 2. Preparation of Free-State Amorphous Form**

10 mg of the compound represented by formula I was added to 1 mL of solvent A. The sample was stirred and slurried at room temperature and then filtered. The filter cake was collected and dried *in vacuo* to give a solid. According to X-ray powder diffraction analysis, the product was a free-state amorphous form. Its XRPD pattern is shown in FIG. 1. Its DSC thermogram showed an endothermic peak at 122.02 °C. Its TGA profile showed a weight loss of 5.06% from 33 °C to 170 °C.

Table 6

| Compound of formula I | Solvent A (1 mL) | Crystal form |
|---|---|---|
| 10 mg | Water | Free-state amorphous form |
| | Methanol | |
| | Ethanol | |
| | Isopropanol | |
| | n-Propanol | |
| | Isopentanol | |
| | Isopropyl acetate | |
| | Methyl tert-butyl ether | |
| | n-Heptane | |
| | Cyclohexane | |
| | Toluene | |

**Example 3. Preparation of Free-state Amorphous form**

The compound represented by formula I was added to solvent B for dissolution. An anti-solvent was then added, resulting in precipitation. The mixture was centrifuged, and drying was then performed *in vacuo* to give a solid. According to X-ray powder diffraction analysis, the product was an amorphous form.

Table 7

<table>
<tr><th>Compound of formula I</th><th>Solvent B</th><th>Anti-solvent</th><th>Crystal form</th></tr>
<tr><td>100 mg</td><td>0.2 mL of dimethyl sulfoxide</td><td>2 mL of water</td><td rowspan="5">Free-state amorphous form</td></tr>
<tr><td rowspan="4">10 mg</td><td>0.1 mL of acetone</td><td rowspan="4">1 mL of water, ethanol, n-heptane, or isopropyl ether</td></tr>
<tr><td>0.1 mL of acetonitrile</td></tr>
<tr><td>0.1 mL of tetrahydrofuran</td></tr>
<tr><td>0.1 mL of dichloromethane</td></tr>
</table>

**Example 4. Preparation of Crystal Form A of Succinate**

10 mg of the compound represented by formula I was added to 0.3 mL of acetone for dissolution, and then 3.26 mg of succinic acid solid was added for dissolution. The mixture was slurried and stirred at room temperature overnight, and centrifuged. Drying was then performed *in vacuo* to give a solid. According to X-ray powder diffraction analysis, the product was defined as crystal form A of succinate. Its XRPD pattern is shown in FIG. 2, with its characteristic peak positions listed in Table 8. Its DSC thermogram showed an endothermic peak at 97.22 °C. Its TGA profile showed a weight loss of 1.51% from 35 °C to 71 °C and a weight loss of 7.23% from 71 °C to 131 °C. Its ion chromatography analysis results showed a succinate ion content of 14.8%.

Table 8

| Peak No. | $2\theta$ value [° or degrees] | D[Å] | Relative intensity% |
|---|---|---|---|
| 1 | 6.170 | 14.31348 | 100.0 |
| 2 | 9.506 | 9.29601 | 0.5 |
| 3 | 11.215 | 7.88346 | 55.6 |
| 4 | 12.380 | 7.14383 | 42.7 |
| 5 | 15.558 | 5.69110 | 37.2 |
| 6 | 18.230 | 4.86250 | 17.3 |
| 7 | 18.884 | 4.69549 | 27.3 |
| 8 | 19.429 | 4.56493 | 19.7 |
| 9 | 21.392 | 4.15031 | 7.0 |
| 10 | 23.609 | 3.76532 | 3.8 |

**Example 5. Preparation of Crystal Form B of Succinate**

10 mg of the compound represented by formula I was added to 0.1 mL of acetone for dissolution, and then 1.63 mg of succinic acid solid was added for dissolution. The mixture was slurried and stirred at room temperature overnight, resulting in the precipitation of a solid. The mixture was centrifuged, and drying was then performed *in vacuo* to give the solid. According to X-ray powder diffraction analysis, the product was defined as crystal form B of succinate. Its XRPD pattern is shown in FIG. 3, with its characteristic peak positions listed in Table 9. Its DSC thermogram showed an endothermic peak at 121.36 °C. Its TGA profile showed a weight loss of 2.19% from 36 °C to 120 °C. Its ion chromatography analysis results showed a succinate ion content of 13.2%.

Table 9

| Peak No. | 20 value [° or degrees] | D[Å] | Relative intensity% |
|---|---|---|---|
| 1 | 6.402 | 13.79430 | 97.2 |
| 2 | 9.400 | 9.40094 | 15.0 |
| 3 | 15.728 | 5.62990 | 17.0 |
| 4 | 17.957 | 4.93571 | 51.7 |

(continued)

| Peak No. | 20 value [° or degrees] | D[Å] | Relative intensity% |
|---|---|---|---|
| 5 | 19.070 | 4.65026 | 100.0 |
| 6 | 21.589 | 4.11298 | 35.4 |

**Example 6. Preparation of Crystal Form C of Succinate**

10 mg of the compound represented by formula I was added to 0.3 mL of tetrahydrofuran for dissolution, and then 3.26 mg of succinic acid solid was added for dissolution. The mixture was slurried and stirred at room temperature overnight, resulting in the precipitation of a solid. The mixture was centrifuged, and drying was then performed *in vacuo* to give the solid. According to X-ray powder diffraction analysis, the product was defined as crystal form C of succinate. Its XRPD pattern is shown in FIG. 4, with its characteristic peak positions listed in Table 10. Its DSC thermogram showed endothermic peaks at 93.71 °C and 118.69 °C. Its TGA profile showed a weight loss of 8.96% from 31 °C to 131 °C. Its ion chromatography analysis results showed a succinate ion content of 19.3%.

Table 10

| Peak No. | 2 value [° or degrees] | D[Å] | Relative intensity% |
|---|---|---|---|
| 1 | 5.555 | 15.89506 | 87.5 |
| 2 | 5.588 | 15.80289 | 91.0 |
| 3 | 9.363 | 9.43823 | 21.5 |
| 4 | 11.165 | 7.91850 | 43.1 |
| 5 | 15.505 | 5.71044 | 8.0 |
| 6 | 18.229 | 4.86269 | 76.6 |
| 7 | 18.668 | 4.74941 | 100.0 |
| 8 | 19.734 | 4.49506 | 43.4 |
| 9 | 21.920 | 4.05165 | 54.6 |
| 10 | 26.943 | 3.30653 | 0.5 |

**Example** 7. **Preparation of Crystal Form D of Succinate**

10 mg of the compound represented by formula I was added to 0.3 mL of acetone for dissolution, and then 1.63 mg of succinic acid solid was added for dissolution. The mixture was slurried and stirred at room temperature overnight, and no precipitation was observed. Subsequently, stirring was performed at a low temperature of 5 °C overnight, resulting in the precipitation of a solid. The mixture was centrifuged, and drying was then performed *in vacuo* to give the solid. According to X-ray powder diffraction analysis, the product was defined as crystal form D of succinate. Its XRPD pattern is shown in FIG. 5, with its characteristic peak positions listed in Table 11. Its DSC thermogram showed an endothermic peak at 132.93 °C. Its TGA profile showed a weight loss of 1.63% from 36 °C to 80 °C and a weight loss of 4.00% from 80 °C to 140 °C. Its ion chromatography analysis results showed a succinate ion content of 12.9%.

Table 11

| Peak No. | 20 value [° or degrees] | D[Å] | Relative intensity% |
|---|---|---|---|
| 1 | 6.878 | 12.84047 | 97.6 |
| 2 | 10.856 | 8.14299 | 24.1 |
| 3 | 11.784 | 7.50365 | 34.9 |
| 4 | 15.639 | 5.66182 | 10.3 |
| 5 | 17.314 | 5.11753 | 10.5 |
| 6 | 18.225 | 4.86384 | 46.2 |
| 7 | 18.597 | 4.76727 | 100.0 |

(continued)

| Peak No. | 2θ value [° or degrees] | D[Å] | Relative intensity% |
|---|---|---|---|
| 8 | 19.103 | 4.64208 | 48.0 |
| 9 | 19.755 | 4.49050 | 24.5 |
| 10 | 20.155 | 4.40213 | 10.8 |
| 11 | 21.477 | 4.13414 | 27.0 |
| 12 | 22.268 | 3.98905 | 9.1 |
| 13 | 24.672 | 3.60555 | 2.6 |
| 14 | 25.655 | 3.46952 | 6.2 |
| 15 | 27.367 | 3.25626 | 19.3 |
| 16 | 28.023 | 3.18155 | 0.2 |
| 17 | 28.715 | 3.10642 | 10.3 |

**Example 8. Preparation of Crystal Form** A **of D-Mandelate**

The compound represented by formula I (100 mg, 125.72 mmol) was dissolved in 8 mL of a mixed solvent of isopropanol and ethyl acetate (V/V = 1/1), and then a solution of D-mandelic acid (19.13 mg, 125.72 μmol) in 0.5 mL of ethyl acetate was added. The mixture was stirred at room temperature for 48 h, resulting in the precipitation of a solid. The reaction mixture was filtered, and the filter cake was collected and dried *in vacuo* to give the title product (100 g, yield: 84.7%). $^1$H NMR nuclear magnetic resonance characterization indicated that the compound represented by formula I formed a salt with D-mandelic acid in a 1:1 ratio.

$^1$H NMR (500 MHz, DMSO) δ 10.76 (d, 1H), 7.64-7.57 (m, 1H), 7.41 (t, 2H), 7.38-7.31 (m, 2H), 7.28 (q, 1H), 7.18-7.11 (m, 2H), 7.08 (d, 1H), 6.91 (s, 1H), 6.81 (d, 1H), 6.70-6.64 (m, 1H), 6.26 (s, 1H), 6.17 (dt, 2H), 5.62 (t, 1H), 5.02-4.98 (m, 1H), 4.31 (t, 1H), 4.04 (q, 1H), 3.45 (d, 1H), 3.39-3.34 (m, 3H), 3.17-3.06 (m, 8H), 3.02 (s, 1H), 2.79-2.70 (m, 1H), 2.58-2.54 (m, 3H), 2.30-2.19 (m, 3H), 2.10 (s, 1H), 1.94-1.85 (m, 2H), 1.79 (s, 2H), 1.74 (s, 2H), 1.58 (dd, 3H), 1.22 (q, 4H), 1.09 (p, 2H).

According to X-ray powder diffraction analysis, the product was defined as crystal form A of D-mandelate, with its 2$\theta$ values listed in Table 12. Its X-ray powder diffraction pattern is shown in FIG. 6.

Its DSC thermogram showed an endothermic peak at 176.10 °C.

Its TGA profile showed a weight loss of 0.91% from 40 °C to 100 °C.

DVS experimental data show that the sample had a hygroscopic weight gain of about 0.833% under normal storage conditions (i.e., 25 °C and 60% RH), a hygroscopic weight gain of about 1.047% under accelerated test conditions (i.e., 70% RH), and a hygroscopic weight gain of about 2.245% under extreme conditions (90% RH). In the process of the humidity changing from 0% to 95% RH, the desorption process of the sample overlapped with its adsorption process. The XRPD pattern shows that the crystal form did not change before and after DVS testing.

Table 12

| Peak No. | 2θ value [° or degrees] | D[Å] | Relative intensity% |
|---|---|---|---|
| 1 | 6.159 | 14.33827 | 18.40 |
| 2 | 6.226 | 14.18374 | 18.20 |
| 3 | 8.371 | 10.55348 | 30.90 |
| 4 | 10.554 | 8.37566 | 53.50 |
| 5 | 13.168 | 6.71805 | 54.30 |
| 6 | 15.728 | 5.63010 | 50.10 |
| 7 | 17.944 | 4.93933 | 100.00 |
| 8 | 19.784 | 4.48381 | 95.50 |
| 9 | 20.381 | 4.35390 | 67.10 |
| 10 | 21.121 | 4.20299 | 96.70 |

(continued)

| Peak No. | 2θ value [° or degrees] | D[Å] | Relative intensity% |
|---|---|---|---|
| 11 | 21.434 | 4.14224 | 62.00 |

**Example 9. Preparation of Crystal Form A of D-Mandelate**

The compound represented by formula I (1.1 g, 1.38 mmol) was dissolved in 40 mL of ethyl acetate, and then a solution of D-mandelic acid (231.46 mg, 1.52 mmol) in 5 mL of ethyl acetate was added, resulting in a clear reaction mixture. The mixture was then stirred at room temperature for 48 h, resulting in the precipitation of a solid. The reaction mixture was filtered, and the filter cake was collected and dried *in vacuo* to give the title product (1.2 g, yield: 92.3%).

$^1$H NMR nuclear magnetic resonance characterization indicated that the compound represented by formula I formed a salt with D-mandelic acid in a 1:1 ratio.

H NMR (500 MHz, DMSO) δ 10.76 (s, 1H), 7.60 (d, 1H), 7.41 (d, 2H), 7.34 (t, 2H), 7.28 (t, 1H), 7.18 -7.11 (m, 2H), 7.07 (t, 1H), 6.91 (t, 1H), 6.81 (d, 1H), 6.67 (dd, 1H), 6.26 (d, 1H), 6.16 (ddd, 2H), 5.62 (d, 1H), 5.00 (s, 1H), 4.31 (ddd, 1H), 4.04 (h, 1H), 3.45 (d, 1H), 3.39-3.33 (m, 3H), 3.18-2.98 (m, 8H), 2.94-2.84 (m, 2H), 2.58 (dt, 1H), 2.49 (s, 2H), 2.16-2.3 (m, 3H), 2.10 (dq, 1H), 1.91-1.72 (m, 6H), 1.63 -1.50 (m, 3H), 1.21 (d, 4H), 1.09 -1.05 (m, 2H). According to X-ray powder diffraction analysis, the product was crystal form A of D-mandelate, with its 2$\theta$ values listed in Table 12.

**Example 10. Preparation of Crystal Form A of D-Mandelate**

100 mg of the compound represented by formula I was added to a mixed solution of 2.5 mL of ethanol and 2.5 mL of ethyl acetate for dissolution, and then 21 mg of D-mandelic acid solid was added for dissolution. The mixture was stirred at room temperature overnight, resulting in precipitation. The mixture was centrifuged, and drying was then performed *in vacuo* to give a solid. According to X-ray powder diffraction analysis, the product was defined as crystal form A of D-mandelate. Its XRPD pattern is shown in FIG. 6, with its characteristic peak positions listed in Table 12.

**Example 11. Preparation of Crystal Form I of Oxalate**

100 mg of the compound represented by formula I was added to 2 mL of acetonitrile solvent, and then 24 mg of oxalic acid solid was added but not dissolved. The mixture was stirred at room temperature overnight to give a white suspended solid. The suspension was centrifuged, and drying was then performed *in vacuo* to give the solid. According to X-ray powder diffraction analysis, the product was defined as crystal form I of oxalate. Its XRPD pattern is shown in FIG. 7, with its characteristic peak positions listed in Table 13. Its DSC thermogram showed an endothermic peak at 136.67 °C. Its TGA profile showed a weight loss of 2.97% from 31 °C to 150 °C. Its ion chromatography analysis results showed an oxalate ion content of 18.7%.

Table 13

| Peak No. | 2θ value [° or degrees] | D[Å] | Relative intensity% |
|---|---|---|---|
| 1 | 4.885 | 18.07327 | 77.4 |
| 2 | 11.219 | 7.88050 | 63.5 |
| 3 | 12.092 | 7.31355 | 100.0 |
| 4 | 14.565 | 6.07687 | 21.6 |
| 5 | 16.383 | 5.40626 | 62.0 |
| 6 | 18.201 | 4.87006 | 25.4 |

**Example 12. Preparation of Amorphous Form of Phosphate**

100 mg of the compound represented by formula I was added to 1 mL of acetone solvent, and then 10 μL of a phosphoric acid solution (85%) was added. The mixture was stirred at room temperature overnight to give a white suspended solid. The suspension was centrifuged, and drying was then performed *in vacuo* to give the solid. According to X-ray powder diffraction analysis, the product was an amorphous form of phosphate. Its XRPD pattern is shown in FIG. 8, and its ion chromatography analysis results showed a phosphate ion content of 12.51%.

**Example 13. Preparation of Crystal Form I of Hydrochloride**

50 mg of the compound represented by formula I was added to 2 mL of acetonitrile solvent, and then 11.5 μL of concentrated hydrochloric acid (36.5%) was added. The mixture was stirred at room temperature overnight to give a white suspended solid. The suspension was centrifuged, and drying was then performed *in vacuo* to give the solid. According to X-ray powder diffraction analysis, the product was defined as crystal form I of hydrochloride. Its XRPD pattern is shown in FIG. 9, with its characteristic peak positions listed in Table 14. Its DSC thermogram showed an endothermic peak at 194.65 °C. Its TGA profile showed a weight loss of 9.15% from 31 °C to 200 °C. Its ion chromatography analysis results showed a chloride ion content of 7.2%.

Table 14

| Peak No. | 2θ value [° or degrees] | D[Å] | Relative intensity% |
|---|---|---|---|
| 1 | 4.891 | 18.05254 | 100.0 |
| 2 | 11.255 | 7.85511 | 10.8 |
| 3 | 16.565 | 5.34732 | 94.7 |
| 4 | 17.947 | 4.93856 | 63.3 |
| 5 | 22.056 | 4.02683 | 8.2 |

**Example** 14. **Preparation of Amorphous Form of Maleate**

100 mg of the compound represented by formula I was added to 2 mL of 2-methyl-tetrahydrofuran solvent, and then 15 mg of maleic acid solid was added. The mixture was stirred at room temperature until it became clear. 8 mL of n-heptane was added, and the mixture was stirred at room temperature overnight and centrifuged. Drying was then performed *in vacuo* to give a solid. According to X-ray powder diffraction analysis, the product was an amorphous form of maleate. Its XRPD pattern is shown in FIG. 10. Its ion chromatography analysis results showed a maleate ion content of 12.74%.

Example 15. **Preparation of Amorphous Form of Fumarate**

10 mg of the compound represented by formula (I) was added to 0.3 mL of tetrahydrofuran solvent, and then 1.5 mg of fumaric acid was added. The mixture was stirred at room temperature until it became clear. 1.0 mL of n-heptane was added, and the mixture was stirred at room temperature overnight and centrifuged. Drying was then performed *in vacuo* to give a solid. According to X-ray powder diffraction analysis, the product was an amorphous form of fumarate. Its XRPD pattern is shown in FIG. 11. Its ion chromatography analysis results showed a fumarate ion content of 12.93%.

Example 16. **Preparation of Amorphous Form of Hydrobromide**

10 mg of the compound represented by formula I was dissolved in 0.3 mL of acetone solvent to give a clear solution. 1.5 μL of hydrobromic acid was added, and the mixture was stirred at room temperature overnight and centrifuged. Drying was then performed *in vacuo* to give a solid. According to X-ray powder diffraction analysis, the product was an amorphous form of hydrobromide. Its XRPD pattern is shown in FIG. 12. Its ion chromatography analysis results showed a bromide ion content of 14.91%.

Example 17. **Preparation of Amorphous Form of Citrate**

10 mg of the compound represented by formula (I) was added to 0.3 mL of tetrahydrofuran solvent, and then 1.5 mg of citric acid was added. The mixture was stirred at room temperature until it became clear. 1.0 mL of n-heptane was added, and the mixture was stirred at room temperature overnight and centrifuged. Drying was then performed *in vacuo* to give a solid. According to X-ray powder diffraction analysis, the product was an amorphous form of citrate. Its XRPD pattern is shown in FIG. 13. Its ion chromatography analysis results showed a citrate ion content of 19.61%.

**Example 18. Preparation of Amorphous Form of Sulfate**

10 mg of the compound represented by formula I was dissolved in 0.3 mL of acetonitrile solvent to give a clear solution. 0.70 μL of concentrated sulfuric acid was added, and the mixture was stirred at room temperature overnight and

centrifuged. Drying was then performed *in vacuo* to give a solid. According to X-ray powder diffraction analysis, the product was an amorphous form of sulfate. Its XRPD pattern is shown in FIG. 14. Its ion chromatography analysis results showed a sulfate ion content of 15.58%.

**Example 19. Preparation of Amorphous Form of Mesylate**

10 mg of the compound represented by formula I was suspended in 0.3 mL of water, and then 0.85 μL of methanesulfonic acid was added. The mixture was left to react at room temperature for 2 h, then stirred at 40 °C overnight, and centrifuged. Drying was then performed *in vacuo* to give a solid. According to X-ray powder diffraction analysis, the product was an amorphous form of mesylate. Its XRPD pattern is shown in FIG. 15.

**Example 20. Preparation of Amorphous Form of Hippurate**

10 mg of the compound represented by formula I was added to 2.48 mg of hippuric acid solid, and the mixture was dissolved in a mixed solvent of 0.1 mL of ethanol and 0.1 mL of ethyl acetate to give a clear solution. 700 μL of MTBE was then added, resulting in precipitation. The mixture was stirred at room temperature overnight and centrifuged. Drying was then performed *in vacuo* to give a solid. According to X-ray powder diffraction analysis, the product was an amorphous form of hippurate. Its XRPD pattern is shown in FIG. 16.

**Example 21. Preparation of Amorphous Form of Benzoate**

10 mg of the compound represented by formula I was added to 1.69 mg of benzoic acid solid, and the mixture was added to a solvent of 0.1 mL of acetonitrile or a mixed solution of 0.1 mL of ethanol and 0.1 mL of ethyl acetate. 700 μL of methyl tert-butyl ether was then added, resulting in precipitation. The mixture was stirred at room temperature overnight and centrifuged. Drying was then performed *in vacuo* to give a solid. According to X-ray powder diffraction analysis, the product was an amorphous form of benzoate. Its XRPD pattern is shown in FIG. 17.

**Example 22. Preparation of Amorphous Form of Benzoate**

10 mg of the compound represented by formula I was added to 1.69 mg of benzoic acid solid, and the mixture was added to 0.3 mL of methyl tert-butyl ether solvent. The resulting mixture was stirred at room temperature overnight and centrifuged. Drying was then performed *in vacuo* to give a solid. According to X-ray powder diffraction analysis, the XRPD pattern showed no significant characteristic peaks, indicating that the product was an amorphous form of benzoate.

**Example 23. Preparation of Amorphous Form of Malate**

10 mg of the compound represented by formula (I) was added to 1.85 mg of malic acid solid, and the mixture was added to a solvent of 0.5 mL of acetonitrile or a mixed solution of 0.1 mL of ethanol and 0.1 mL of ethyl acetate. 700 μL of methyl tert-butyl ether was then added, resulting in precipitation. The mixture was stirred at room temperature overnight and centrifuged. Drying was then performed *in vacuo* to give a solid. According to X-ray powder diffraction analysis, the product was an amorphous form of malate. Its XRPD pattern is shown in FIG. 18.

**Example 24. Preparation of Amorphous Form of Malate**

10 mg of the compound represented by formula (I) was added to 1.85 mg of malic acid solid, and the mixture was added to 0.3 mL of tetrahydrofuran solvent. 700 μL of methyl tert-butyl ether was then added, resulting in precipitation. The mixture was stirred at room temperature overnight and centrifuged. Drying was then performed *in vacuo* to give a solid. According to X-ray powder diffraction analysis, the product was an amorphous form of malate. Its XRPD pattern is shown in FIG. 18.

**Example 25. Preparation of Amorphous Form of Acetate**

10 mg of the compound represented by formula (I) was added to 11.5 μL of a 1.2 M acetic acid-ethanol solution, and the mixture was added to 0.3 mL of acetonitrile or ethyl acetate solvent to give a clear solution. 700 μL of methyl tert-butyl ether was then added, resulting in precipitation. The mixture was stirred at room temperature overnight and centrifuged. Drying was then performed *in vacuo* to give a solid. According to X-ray powder diffraction analysis, the product was an amorphous form of acetate. Its XRPD pattern is shown in FIG. 19.

**Example 26. Preparation of Amorphous Form of Acetate**

10 mg of the compound represented by formula I was added to 11.5 μL of a 1.2 M acetic acid-ethanol solution, and the mixture was added to 0.3 mL of a methyl tert-butyl ether solution. The mixture was stirred at room temperature overnight and centrifuged. Drying was then performed *in vacuo* to give a solid. According to X-ray powder diffraction analysis, the XRPD pattern showed no significant characteristic peaks, indicating that the product was an amorphous form of acetate.

**Example 28. Stability Study of Amorphous Form**

Influencing factor stability study: Samples of the free-state form were taken, spread in open containers, and left to stand under high-temperature (40 °C and 60 °C) and high-humidity (RH 75% and RH 92.5%) conditions for 30 days to study its stability.

Table 15

| Condition | Time (days) | Purity (%) | Crystal form |
|---|---|---|---|
| Initial | 0 | 98.8 | Amorphous form |
| 40 °C | 5 | 98.7 | No change |
| | 10 | 98.6 | No change |
| | 30 | 98.6 | No change |
| 60 °C | 5 | 98.6 | No change |
| | 10 | 98.6 | No change |
| | 30 | 98.4 | No change |
| 75% RH | 5 | 98.7 | No change |
| | 10 | 98.7 | No change |
| | 30 | 98.7 | No change |
| 92.5% RH | 5 | 98.7 | No change |
| | 10 | 98.7 | No change |
| | 30 | 98.6 | No change |

The results of the influencing factor experiment show that: the free-state amorphous form exhibited good chemical stability under high-temperature and high-humidity conditions. Long-term/accelerated stability study: Samples of the free-state form were sealed in aluminum foil bags and left to stand under 25 °C/60% RH and 40 °C/75% RH conditions to study its stability. The results are shown below.

Table 16

| Storage conditions | Purity (%) | | | | | Crystal form |
|---|---|---|---|---|---|---|
| | Initia 1 | 1 month | 2 months | 3 months | 6 months | Amorphous form |
| 25 °C/60% RH | 98.8 | 98.7 | 98.5 | 98.5 | 98.5 | No change |
| 40 °C/75% RH | | 98.7 | 98.4 | 98.4 | 98.3 | No change |

The experimental results show that: standing under long-term accelerated conditions for 6 months, the free-state amorphous form exhibited good physical and chemical stability.

**Example 29. Stability Study of Salt Forms**

Influencing factor stability study of salt forms: Samples of the salt forms were taken, spread in open containers, and left to stand under high-temperature (40 °C and 60 °C) and high-humidity (RH 75%) conditions for 30 days to study their stability.

Table 17

| Condition | Time (days) | Purity (%) | Crystal form |
| --- | --- | --- | --- |
| Initial | 0 | 98.1 | Crystal form I of hydrochloride |
| 40 °C | 5 | 97.5 | No change |
| | 10 | 96.9 | No change |
| | 30 | 95.3 | - |
| 60 °C | 5 | 97.2 | No change |
| | 10 | 96.5 | No change |
| | 30 | 94.5 | - |
| 75% RH | 5 | 97.5 | No change |
| | 10 | 97.1 | No change |
| | 30 | 96.5 | -- |

Table 18

| Condition | Time (days) | Purity (%) | Crystal form |
| --- | --- | --- | --- |
| Initial | 0 | 98.8 | Crystal form B of succinate |
| 40 °C | 5 | 98.5 | No change |
| | 10 | 98.3 | No change |
| | 30 | 97.8 | No change |
| 60 °C | 5 | 98.4 | No change |
| | 10 | 97.9 | No change |
| | 30 | 97.0 | No change |
| 75% RH | 5 | 98.6 | No change |
| | 10 | 98.4 | No change |
| | 30 | 98.4 | No change |

Table 19

| Condition | Time (days) | Purity (%) | Crystal form |
| --- | --- | --- | --- |
| Initial | 0 | 98.6 | Crystal form A of mandelate |
| 40 °C | 5 | 98.6 | No change |
| | 10 | 98.6 | No change |
| | 30 | 98.4 | No change |
| 60 °C | 5 | 98.4 | No change |
| | 10 | 98.5 | No change |
| | 30 | 98.2 | No change |
| 75% RH | 5 | 98.5 | No change |
| | 10 | 98.5 | No change |
| | 30 | 98.5 | No change |

Long-term/accelerated stability of salt forms: Samples were sealed in aluminum foil bags and left to stand under 25 °C/60% RH and 40 °C/75% RH conditions to study their stability. The results are shown below.

Table 20

| | Storage conditions | Purity (%) | | | | | Crystal form |
|---|---|---|---|---|---|---|---|
| Crystal form B of succinate | | Initial | 1 month | 2 months | 3 months | 6 months | |
| | 25 °C/60% RH | 98.7 | 98.5 | 98.5 | 98.7 | 98.6 | No change |
| | 40 °C/75% RH | | 98.5 | 98.5 | 98.5 | 98.3 | No change |
| | Storage conditions | Purity (%) | | | | | Crystal form |
| Crystal form A of mandelate | | Initial | 1 month | 2 months | 3 months | 6 months | |
| | 25 °C/60% RH | 99.4 | 99.4 | 99.3 | 99.3 | 99.3 | No change |
| | 40 °C/75% RH | | 99.4 | 99.3 | 99.3 | 98.9 | No change |
| | Storage conditions | Purity (%) | | | | | Crystal form |
| Amorphous form of maleate | | Initial | 1 month | 2 months | 3 months | 6 months | |
| | 25 °C/60% RH | 98.9 | 98.9 | 98.9 | 98.9 | 98.7 | No change |
| | 40 °C/75% RH | | 98.7 | 98.6 | 98.3 | 94.8 | No change |

The experimental results show that: standing under long-term accelerated conditions for 6 months, the crystal form B of succinate and the crystal form A of mandelate exhibited good chemical and physical stability; standing under long-term conditions for 6 months, the amorphous form of maleate exhibited good chemical and physical stability.

## Claims

1. An amorphous form of the compound represented by formula I.

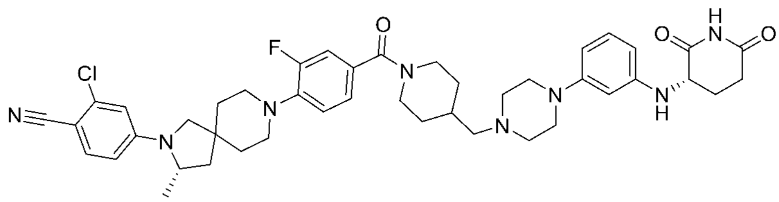

I

2. The amorphous form according to claim 1, wherein an XRPD pattern of the amorphous form has no sharp peaks and preferably has non-sharp peaks.

3. A pharmaceutically acceptable salt of the compound represented by formula I, being selected from the group consisting of succinate, mandelate, oxalate, phosphate, hydrochloride, maleate, fumarate, hydrobromide, citrate, sulfate, mesylate, hippurate, benzoate, malate, and acetate, preferably succinate and mandelate, and more preferably succinate and D-mandelate.

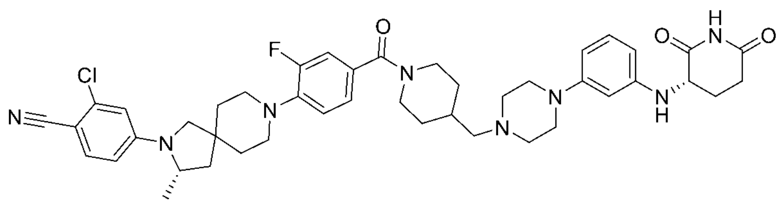

I

4. The pharmaceutically acceptable salt of the compound represented by formula I according to claim 3, wherein a chemical ratio of the compound represented by formula I to an acid molecule or an acid radical is 2:0.5-1:3; preferably, the chemical ratio of the compound represented by formula I to the acid molecule or the acid radical is selected from the group consisting of 2:1-1:3; more preferably, the chemical ratio of the compound represented by formula I to the acid molecule or the acid radical is selected from the group consisting of 1:0.5, 1:1, 1:1.5, and 1:2.

5. A succinate of the compound represented by formula I, wherein a chemical ratio of the compound of formula I to succinate ion or succinic acid is about 2:1-1:3, preferably 1:1 or 1:2.

6. A crystal form B of succinate of the compound represented by formula I, wherein an X-ray powder diffraction pattern expressed in terms of 2$\theta$ angles, which are diffraction angles, of the crystal form has characteristic peaks at 6.402, 9.400, 15.728, 17.957, and 19.070, preferably at 6.402, 9.400, 15.728, 17.957, 19.070, and 21.589; more preferably, the X-ray powder diffraction pattern expressed in terms of 2$\theta$ angles, which are diffraction angles, of the crystal form is shown in FIG. 3.

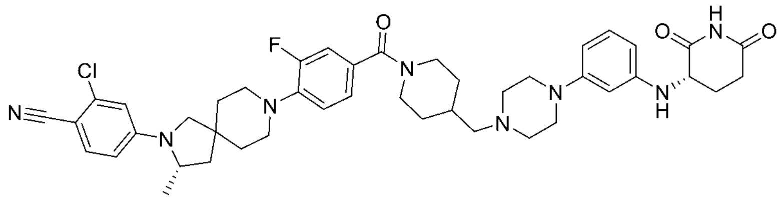

I

7. A preparation method for the crystal form according to claim 6, comprising: mixing the compound represented by formula I with a solvent and succinic acid, and crystallizing to give the crystal form B of succinate, wherein the solvent is selected from the group consisting of a ketone solvent; preferably, the ketone solvent is acetone.

8. A D-mandelate of the compound represented by formula I, wherein a chemical ratio of the compound of formula I to mandelate ion or mandelic acid is about 1:1.

9. A crystal form A of D-mandelate of the compound represented by formula I, wherein an X-ray powder diffraction pattern expressed in terms of 2$\theta$ angles, which are diffraction angles, of the crystal form has characteristic peaks at 10.554, 13.168, 15.728, 17.944, and 19.784, preferably at 6.159, 8.371, 10.554, 13.168, 15.728, 17.944, 19.784, and 20.381, and more preferably at 6.159, 8.371, 10.554, 13.168, 15.728, 17.944, 19.784, 20.381, 21.121, and 21.434; further preferably, the X-ray powder diffraction pattern expressed in terms of 2$\theta$ angles, which are diffraction angles, of the crystal form is shown in FIG. 6.

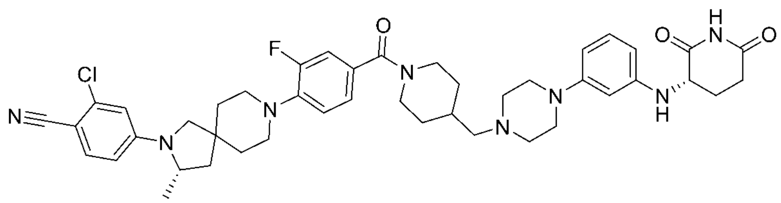

I

10. A preparation method for the crystal form according to claim 9, comprising: mixing the compound represented by formula I with a solvent and D-mandelic acid, and crystallizing to give the crystal form A of D-mandelate, wherein the solvent is selected from the group consisting of one or more of isopropanol, ethanol, and ethyl acetate, and preferably, the solvent is selected from the group consisting of isopropanol/ethyl acetate, ethanol/ethyl acetate, and ethyl acetate.

11. A pharmaceutical composition, comprising: the amorphous form according to any one of claims 1-2, the pharmaceutically acceptable salt according to claims 3-5 and 8, the crystal form according to any one of claims 6 and 9, or the crystal form prepared by the preparation method according to any one of claims 7 and 10; and optionally a pharmaceutically acceptable excipient.

12. A preparation method for the pharmaceutical composition according to claim 11, comprising a step of mixing the amorphous form according to any one of claims 1-2, the pharmaceutically acceptable salt according to claims 3-5 and 8, the crystal form according to any one of claims 6 and 9, or the crystal form prepared by the preparation method according to any one of claims 7 and 10 with the pharmaceutically acceptable excipient.

13. Use of the amorphous form according to any one of claims 1-2, the pharmaceutically acceptable salt according to claims 3-5 and 8, the crystal form according to any one of claims 6 and 9, the crystal form prepared by the preparation

method according to any one of claims 7 and 10, or the pharmaceutical composition according to claim 11 in the manufacture of a medicament for regulating the ubiquitination and degradation of the androgen receptor (AR) protein.

**14.** Use of the amorphous form according to any one of claims 1-2, the pharmaceutically acceptable salt according to claims 3-5 and 8, the crystal form according to any one of claims 6 and 9, the crystal form prepared by the preparation method according to any one of claims 7 and 10, or the pharmaceutical composition according to claim 11 in the manufacture of a medicament for treating and/or preventing an androgen receptor-mediated or -dependent disease or disorder, wherein the androgen receptor-mediated or -dependent disease or disorder is preferably selected from the group consisting of a tumor, male sexual dysfunction, and Kennedy's disease.

**15.** The use according to claim 14, wherein the androgen receptor-mediated or -dependent disease or disorder is selected from the group consisting of prostate cancer, prostatic hyperplasia, hirsutism, alopecia, anorexia nervosa, breast cancer, acne, male sexual dysfunction, Kennedy's disease, and AIDS, preferably prostate cancer, and more preferably hormone-sensitive prostate cancer or hormone-refractory prostate cancer.

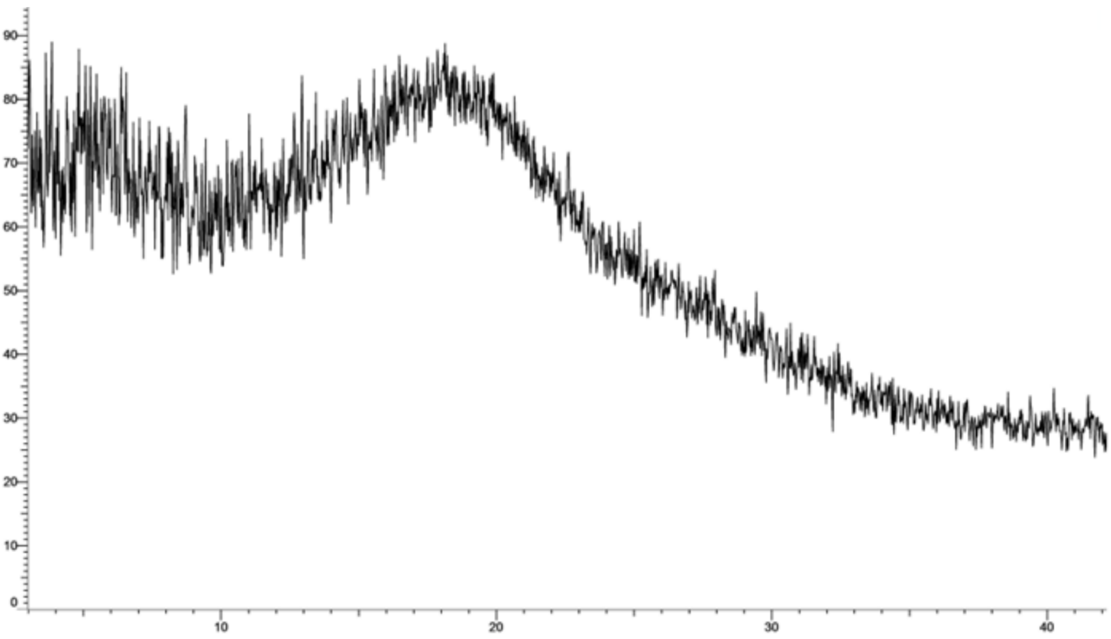
0
10
20
30
40
50
60
70
80
90
10
20
30
40

FIG. 1

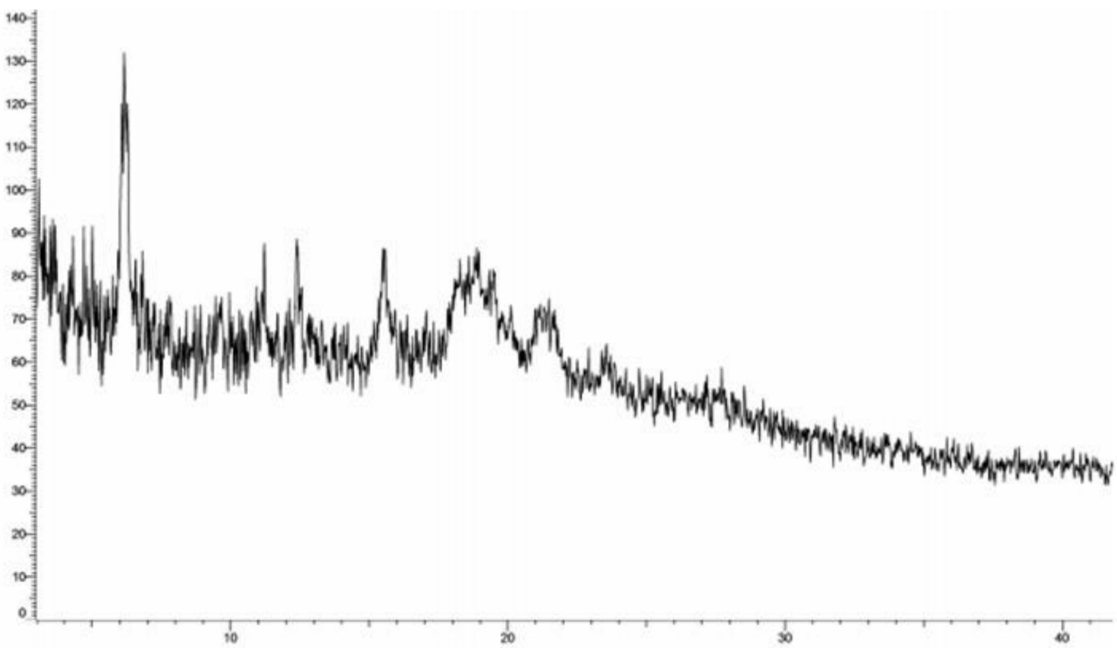
0
10
20
30
40
50
60
70
80
90
100
110
120
130
140
10
20
30
40

FIG. 2

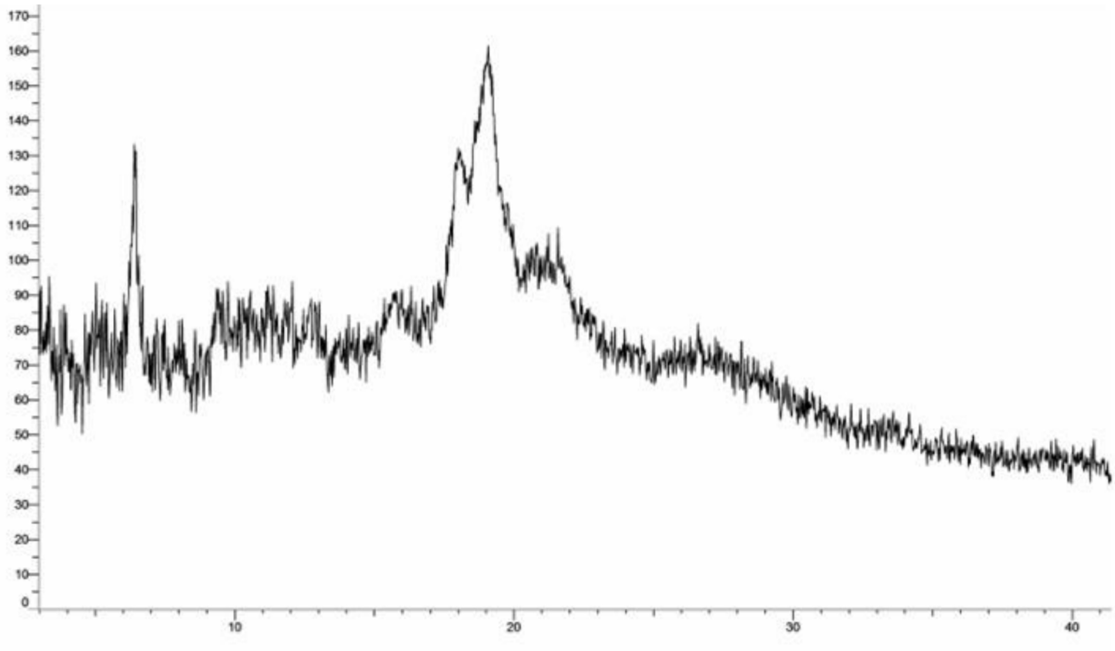
170
160
150
140
130
120
110
100
90
80
70
60
50
40
30
20
10
0
10
20
30
40

FIG. 3

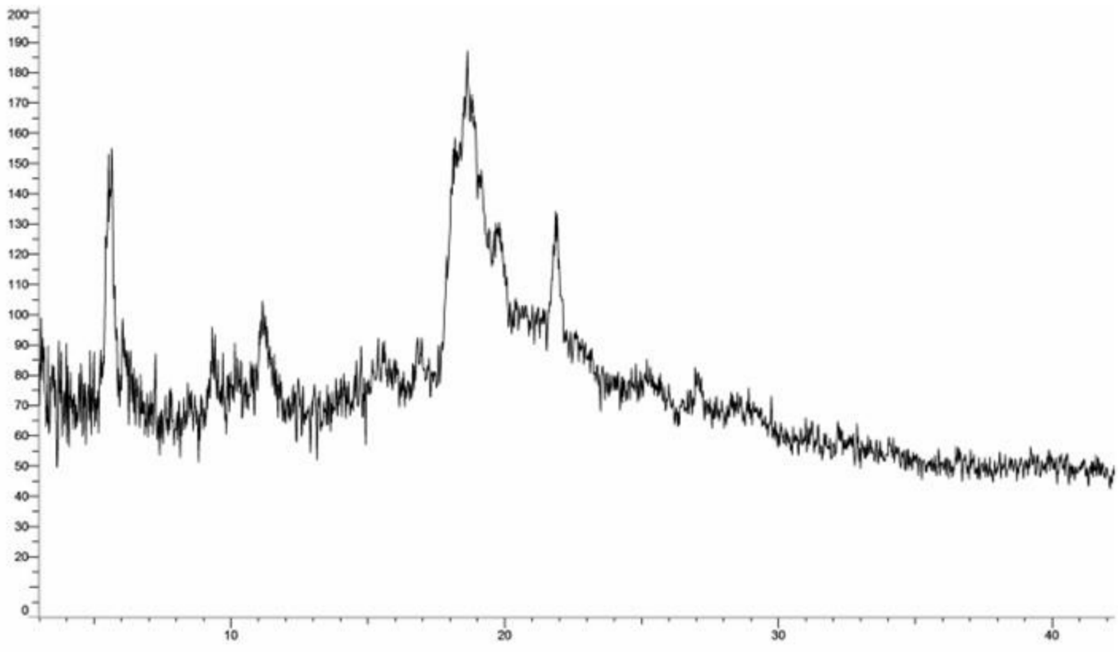
200
190
180
170
160
150
140
130
120
110
100
90
80
70
60
50
40
30
20
0
10
20
30
40

FIG. 4

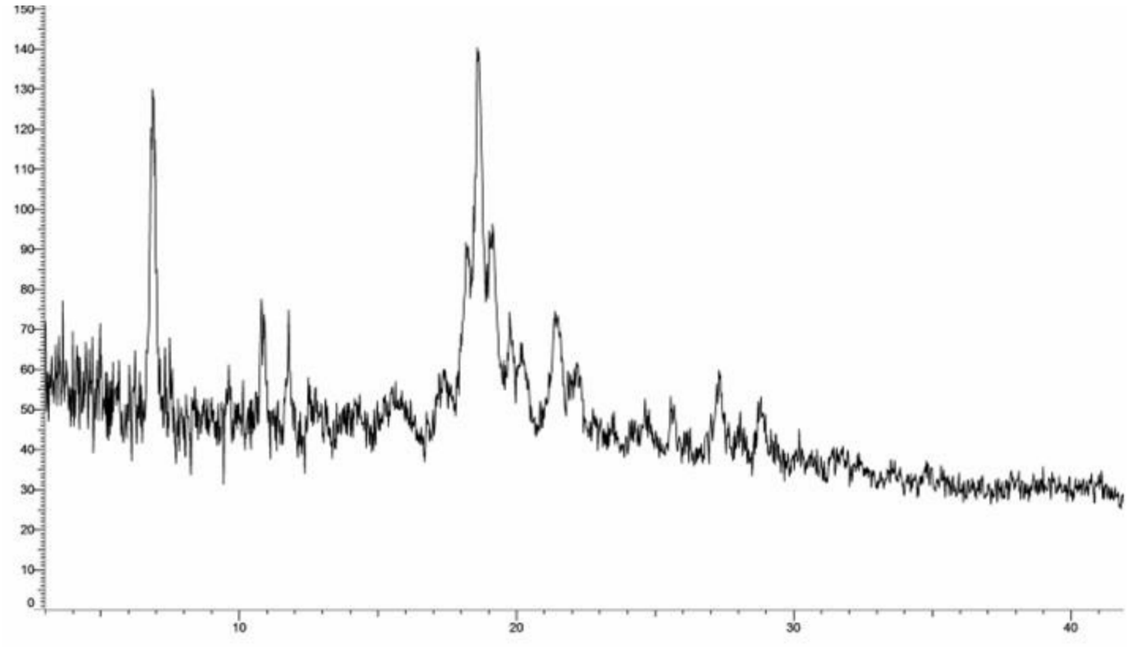
150
140
130
120
110
100
90
80
70
60
50
40
30
20
10
0
10
20
30
40

FIG. 5

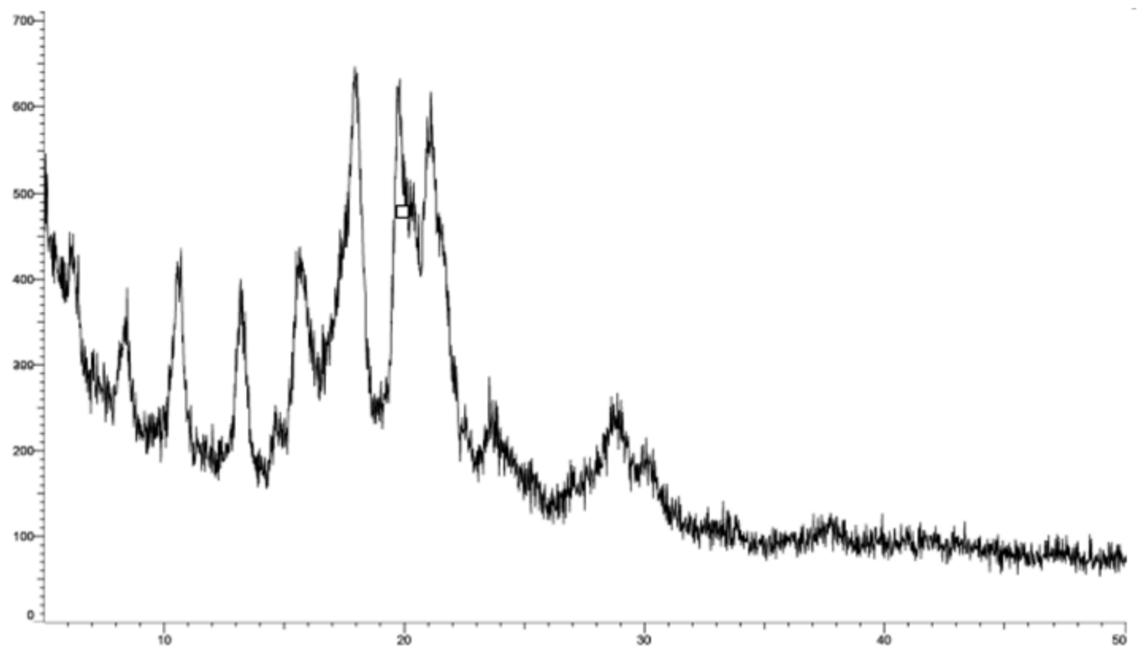
700
600
500
400
300
200
100
0
10
20
30
40
50

FIG. 6

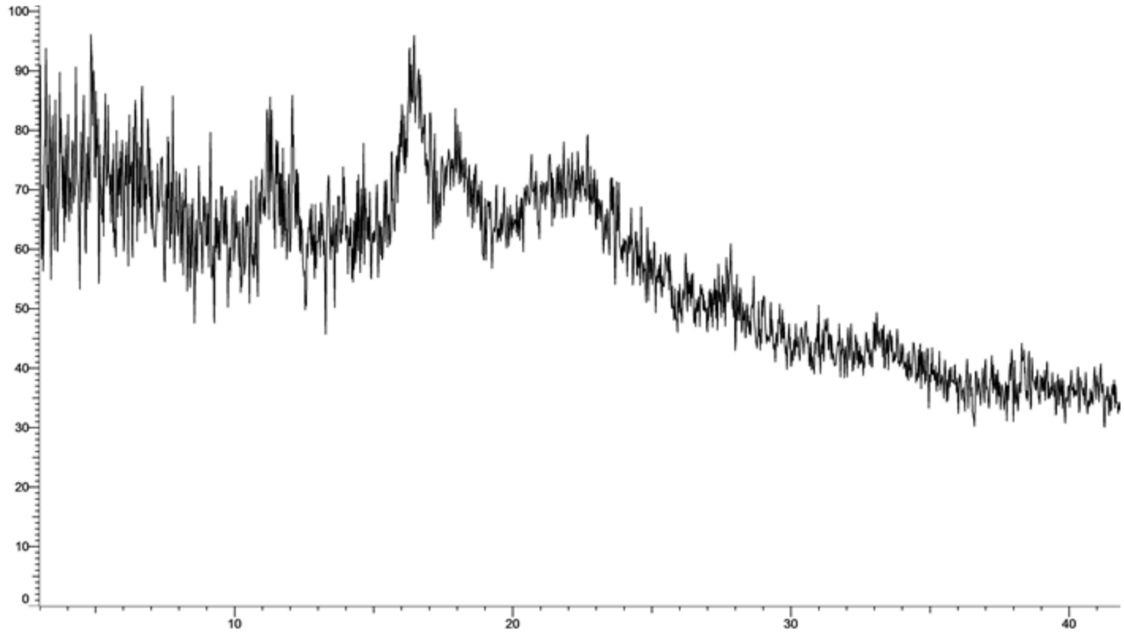
100
90
80
70
60
50
40
30
20
10
0
10
20
30
40

FIG. 7

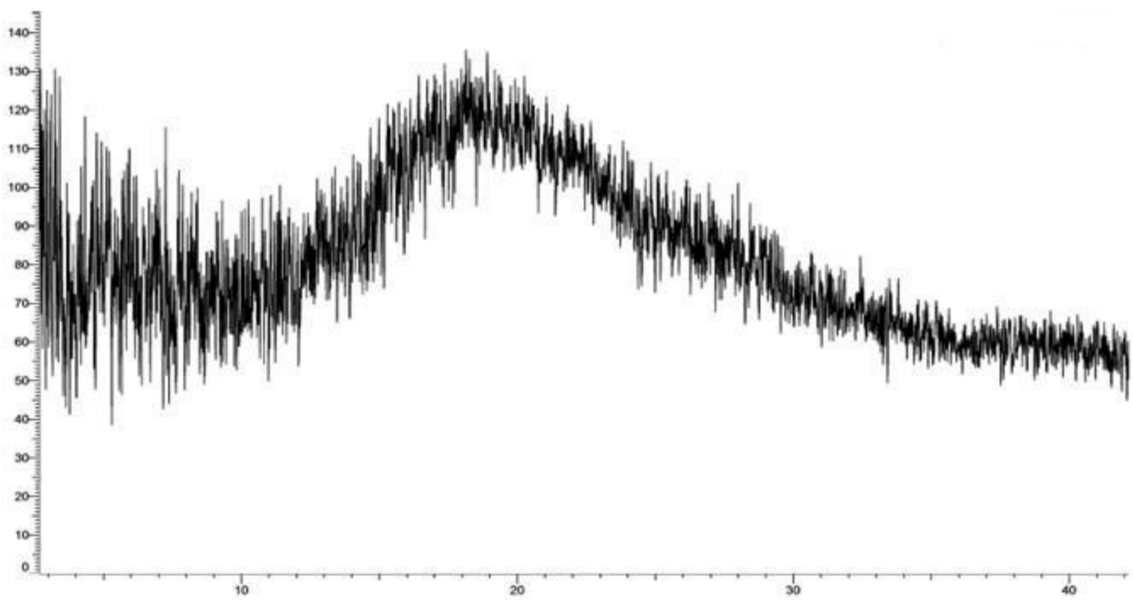
140
130
120
110
100
90
80
70
60
50
40
30
20
10
0
10
20
30
40

FIG. 8

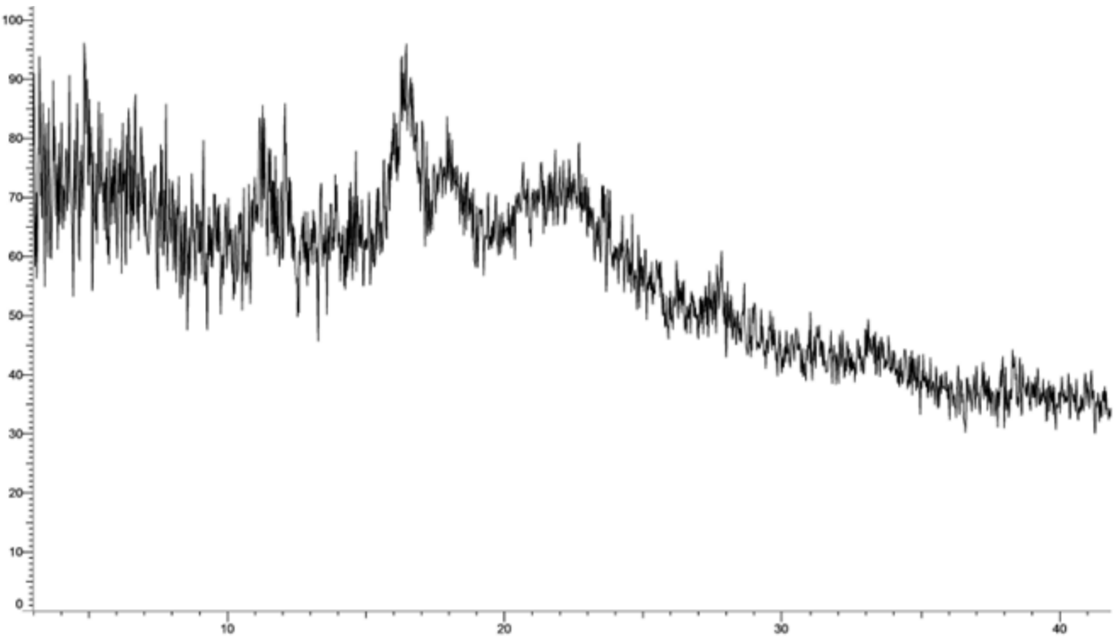
100
90
80
70
60
50
40
30
20
10
0
10
20
30
40

FIG. 9

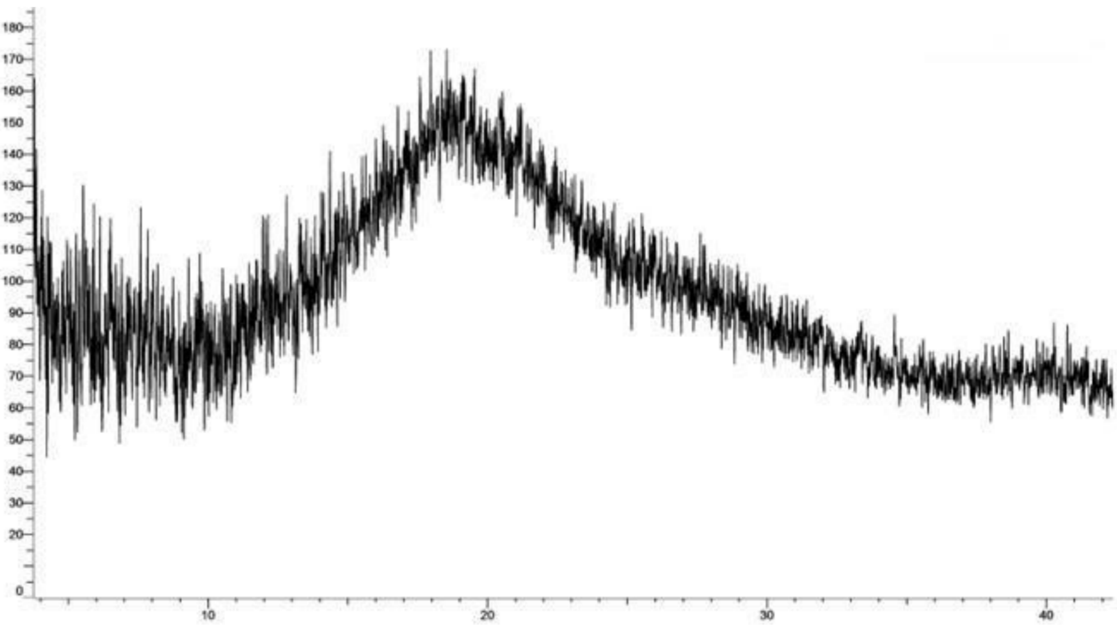
180
170
160
150
140
130
120
110
100
90
80
70
60
50
40
30
20
0
10
20
30
40

FIG. 10

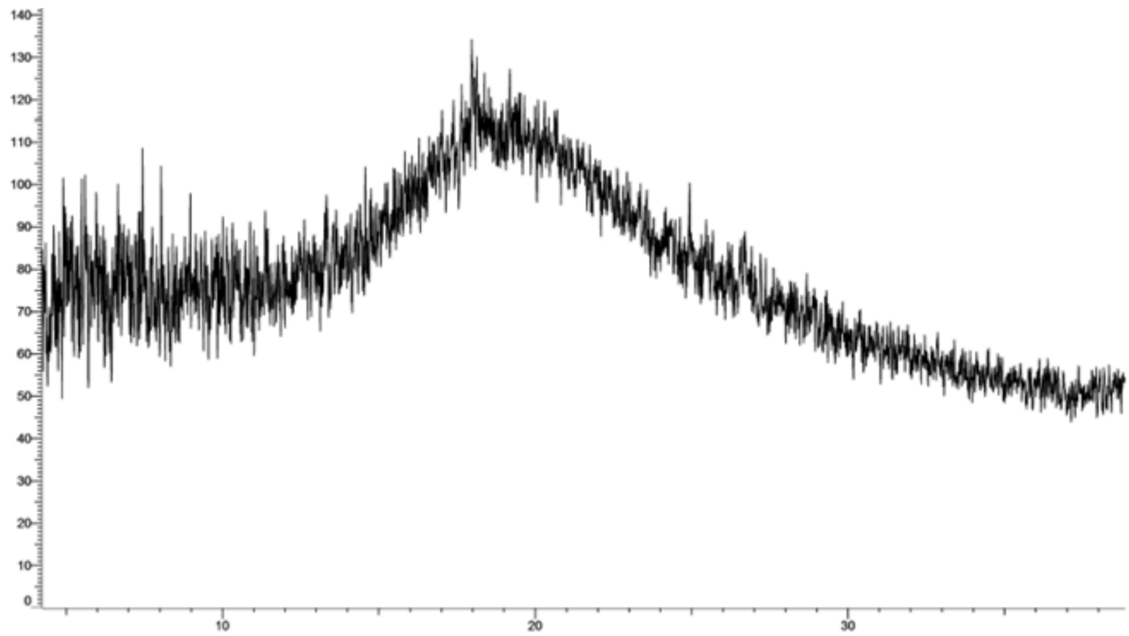
140
130
120
110
100
90
80
70
60
50
40
30
20
10
0
10
20
30

FIG. 11

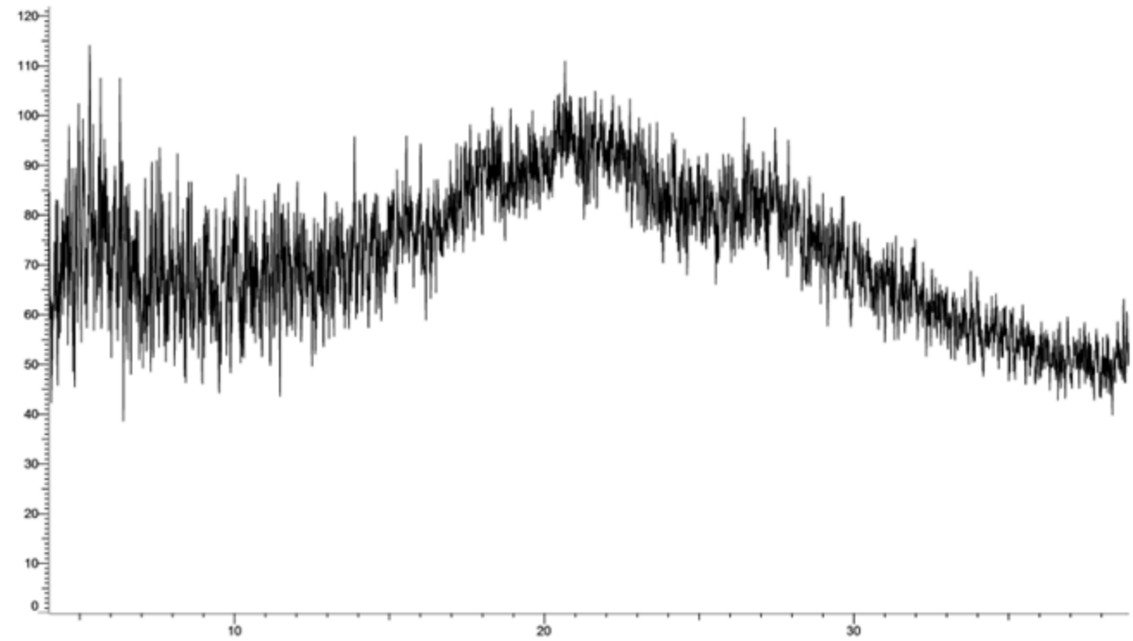
120
110
100
90
80
70
60
50
40
30
20
10
0
10
20
30

FIG. 12

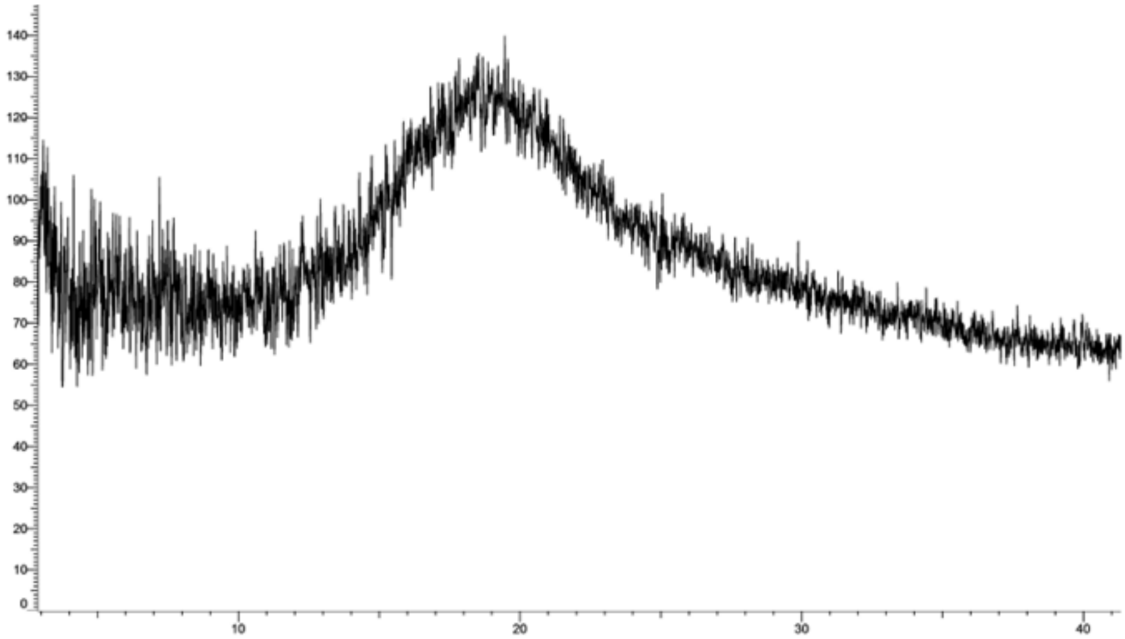
140
130
120
110
100
90
80
70
60
50
40
30
20
10
0
10
20
30
40

FIG. 13

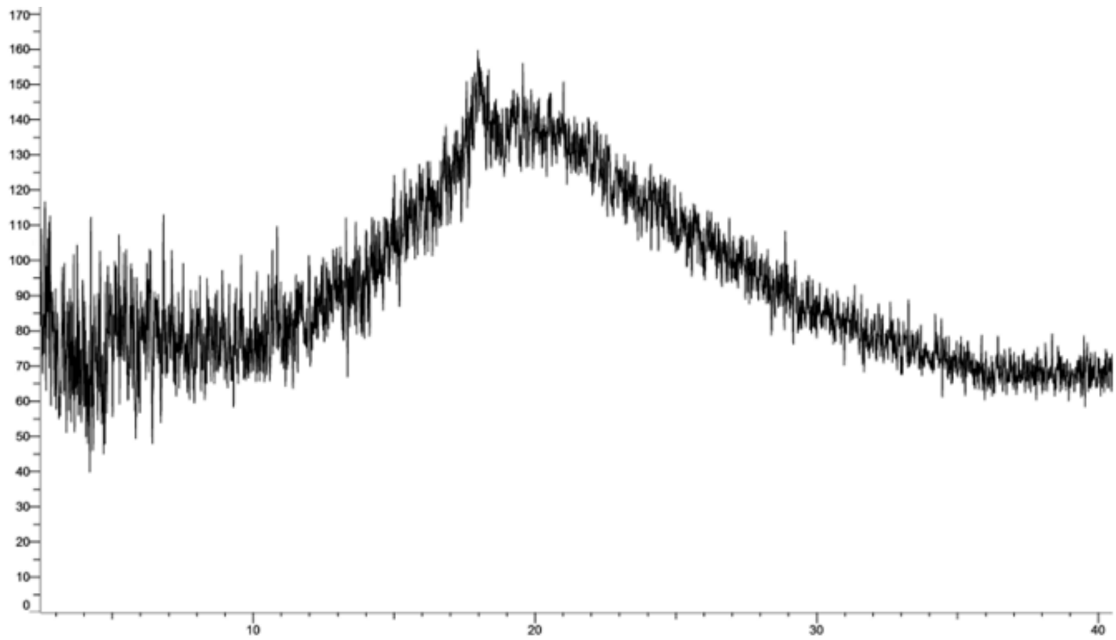
170
160
150
140
130
120
110
100
90
80
70
60
50
40
30
20
10
0
10
20
30
40

FIG. 14

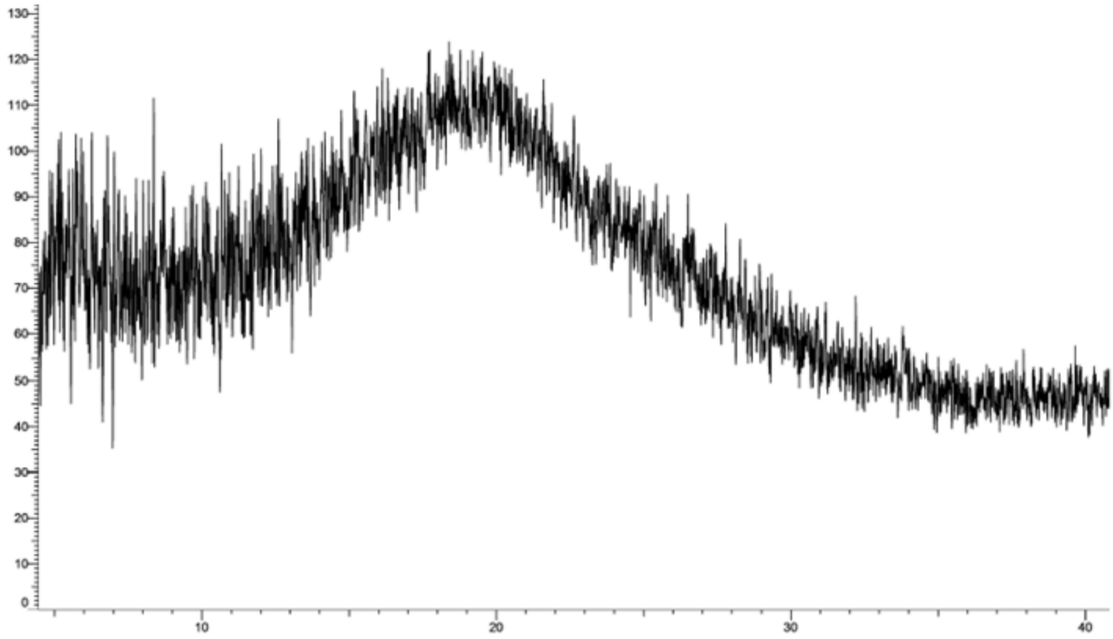
130
120
110
100
90
80
70
60
50
40
30
20
10
0
10
20
30
40

FIG. 15

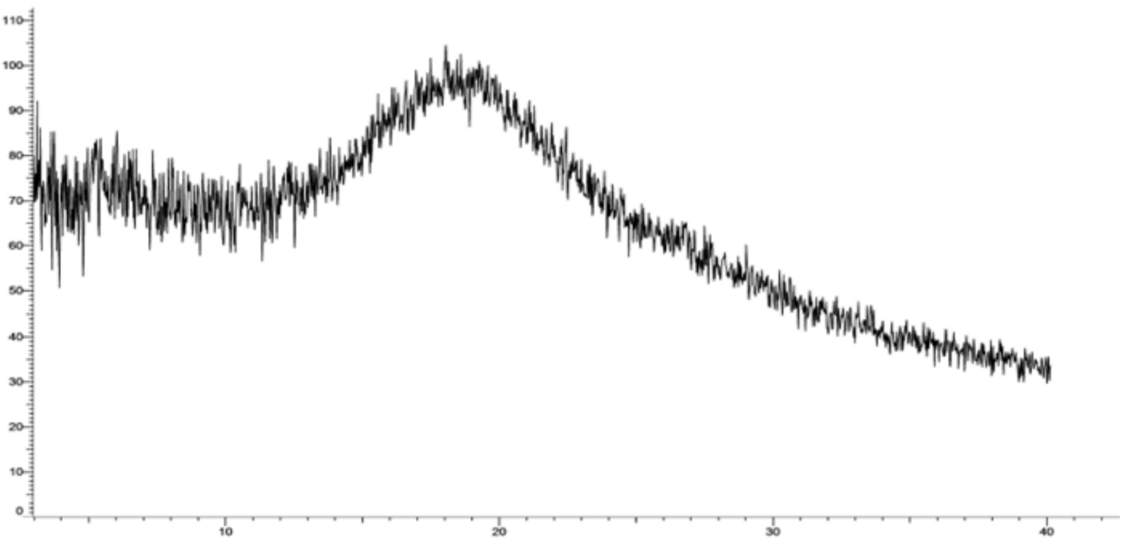
110
100
90
80
70
60
50
40
30
20
10
0
10
20
30
40

FIG. 16

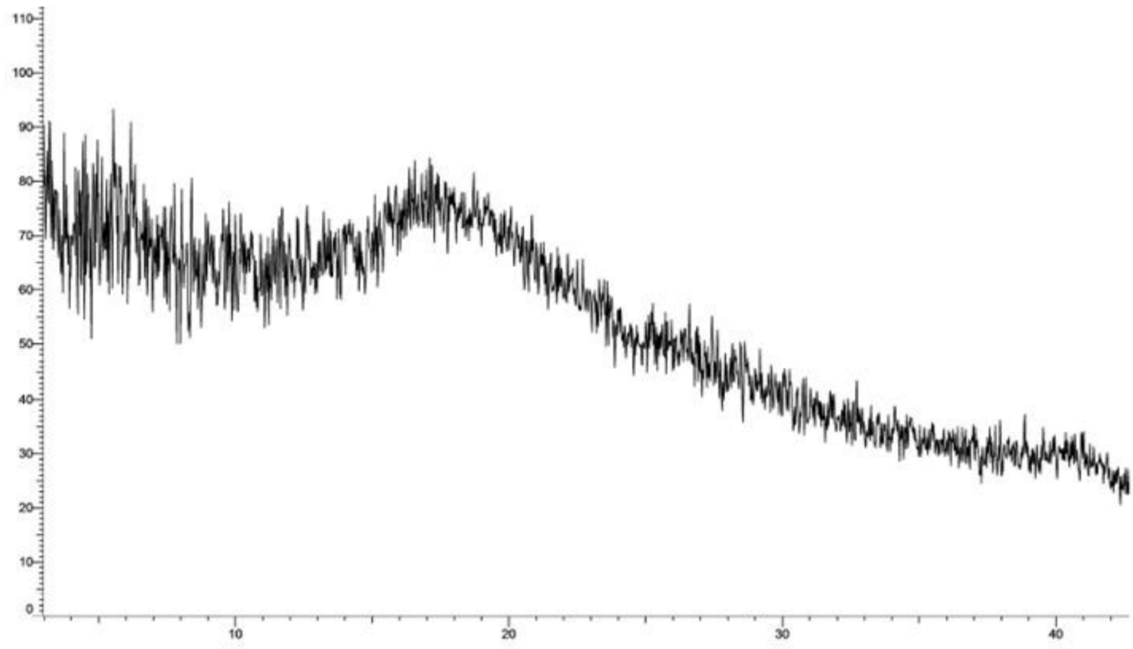
110
100
90
80
70
60
50
40
30
20
10
0
10
20
30
40

FIG. 17

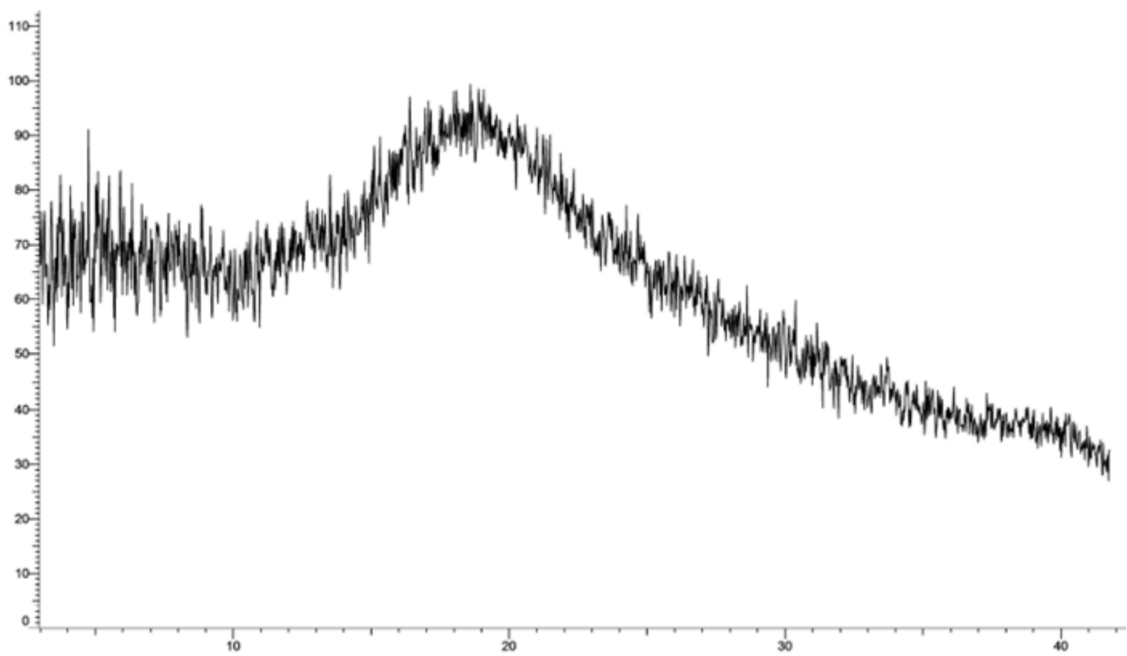
110
100
90
80
70
60
50
40
30
20
10
0
10
20
30
40

FIG. 18

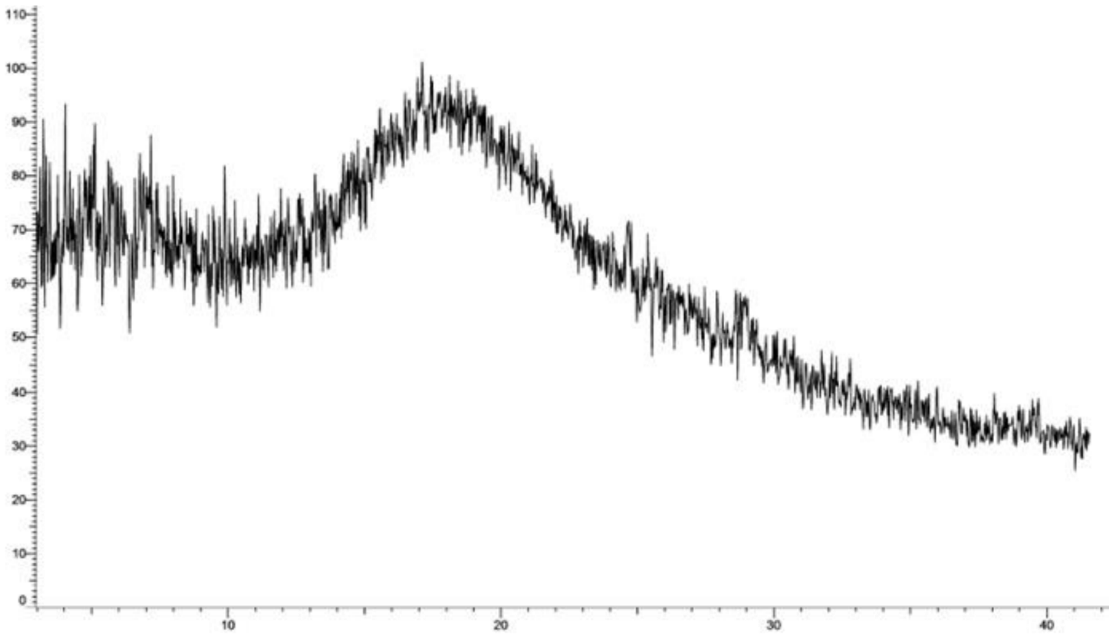


FIG. 19

## INTERNATIONAL SEARCH REPORT

International application No. **PCT/CN2024/095156**

**A. CLASSIFICATION OF SUBJECT MATTER**

C07D471/10(2006.01)i； A61K31/497(2006.01)i； A61P13/08(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： C07D A61K A61P

Documentation searched other than minimum documentation to the extent that such documents are included in the fields searched

Electronic data base consulted during the international search (name of data base and, where practicable, search terms used)

CNABS; CNTXT; DWPI; CNKI; ENTXT; ENTXTC; 超星读秀, DUXIU; ISI_Web of Science; STN-REG; STN-CAPLUS: 江苏恒瑞医药股份有限公司, 上海恒瑞医药有限公司, 周先强, 尚婷婷, 王林, 杜振兴, 杨俊然, 二氧代哌啶, 雄激素受体, 肿瘤, 癌症, 艾滋病, 泛素连接酶, dioxopiperidine, androgen receptor, AR, cancer, AIDS, ubiquitin ligase

**C. DOCUMENTS CONSIDERED TO BE RELEVANT**

| Category* | Citation of document, with indication, where appropriate, of the relevant passages | Relevant to claim No. |
|---|---|---|
| A | CN 115023267 A (THE REGENTS OF THE UNIVERSITY OF MICHIGAN) 06 September 2022 (2022-09-06)<br>abstract, claims 112, 113, 116, and 117, and description, embodiment 47 | 1-15 |
| A | CN 115175901 A (ARVINAS OPERATIONS, INC.) 11 October 2022 (2022-10-11)<br>entire document | 1-15 |
| A | WO 2022127904 A1 (JIANGSU HENGRUI PHARMACEUTICALS CO., LTD. et al.) 23 June 2022 (2022-06-23)<br>entire document | 1-15 |
| A | WO 2021058017 A1 (BEIGENE, LTD. et al.) 01 April 2021 (2021-04-01)<br>entire document | 1-15 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 July 2024** | **24 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/095156**

| Patent document cited in search report | | | Publication date (day/month/year) | Patent family member(s) | | | Publication date (day/month/year) |
|---|---|---|---|---|---|---|---|
| CN | 115023267 | A | 06 September 2022 | EP | 4031241 | A1 | 27 July 2022 |
| | | | | JP | 2022548775 | A | 21 November 2022 |
| | | | | WO | 2021055756 | A1 | 25 March 2021 |
| | | | | AU | 2020348849 | A1 | 07 April 2022 |
| | | | | CA | 3155010 | A1 | 25 March 2021 |
| | | | | US | 2022380368 | A1 | 01 December 2022 |
| CN | 115175901 | A | 11 October 2022 | US | 2024131023 | A1 | 25 April 2024 |
| | | | | KR | 20220119094 | A | 26 August 2022 |
| | | | | WO | 2021127443 | A1 | 24 June 2021 |
| | | | | IL | 293961 | A | 01 August 2022 |
| | | | | AU | 2020405129 | A1 | 23 June 2022 |
| | | | | JP | 2023507570 | A | 24 February 2023 |
| | | | | EP | 4077309 | A1 | 26 October 2022 |
| | | | | BR | 112022012110 | A2 | 13 December 2022 |
| | | | | MX | 2022007678 | A | 19 September 2022 |
| | | | | US | 2021196710 | A1 | 01 July 2021 |
| | | | | US | 11883393 | B2 | 30 January 2024 |
| | | | | CA | 3165168 | A1 | 24 June 2021 |
| | | | | TW | 202136240 | A | 01 October 2021 |
| | | | | IN | 202247036002 | A | 01 July 2022 |
| | | | | SA | 522433088 | A | 19 January 2023 |
| | | | | VN | 93251 | A | 27 March 2023 |
| | | | | HK | 40078792 | A0 | 06 April 2023 |
| | | | | CN | 115175901 | B | 22 March 2024 |
| | | | | CN | 118059104 | A | 24 May 2024 |
| WO | 2022127904 | A1 | 23 June 2022 | TW | 202241879 | A | 01 November 2022 |
| | | | | CN | 116615418 | A | 18 August 2023 |
| WO | 2021058017 | A1 | 01 April 2021 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023105921952
- CN 2022134333 W
- WO 2018237026 A1
- WO 2021055756 A1
- WO 2018071606 A1

### Non-patent literature cited in the description

- *Cell Biochem Funct.*, 2019, vol. 37, 21-30
- *Science*, 2010, vol. 327, 1345
- *Science*, 2014, vol. 343, 301
- *Science*, 2014, vol. 343, 305
- General Rule 9103, Guidelines for Hygroscopicity Trials for Pharmaceuticals. Chinese Pharmacopoeia. 2015, vol. IV